(19) Europäisches Patentamt
European Patent Office
Office européen des brevets

(11) Publication number: **0 013 143**
**A2**

# (12) EUROPEAN PATENT APPLICATION

(21) Application number: 79302986.9

(22) Date of filing: 20.12.79

(51) Int. Cl.[3]: C 07 D 239/42
A 01 N 43/54

(30) Priority: 22.12.78 AU 7204/78
28.03.79 AU 8210/79

(43) Date of publication of application:
09.07.80 Bulletin 80/14

(84) Designated Contracting States:
BE CH DE FR GB IT NL

(71) Applicant: ICI AUSTRALIA LIMITED
ICI House 1 Nicholson Street P.O.Box 4311
Melbourne Victoria 3001(AU)

(72) Inventor: Serban, Alexander
3 Maple Court
Doncaster, Victoria 3108(AU)

(72) Inventor: Watson, Keith Geoffrey
36 Medway Street
Box Hill North, Victoria 3129(AU)

(72) Inventor: Warner, Richard Burridge
8 Baron Court
Ringwood,Victoria 3134(AU)

(74) Representative: Fawcett, Richard Fennelly et al,
Imperial Chemical Industries Limited Legal Department:
Patents Thames House North Millbank
London SW1P 4QG(GB)

## (54) Substituted anilinopyrimidines, compositions and use thereof as herbicides, processes for their preparation and intermediates for use therein.

(57) The invention concerns novel compounds of the formula I

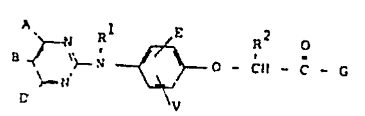

I

The compounds are herbicides and in further embodiments the invention provides herbicidal compositions containing as active ingredient a compound of formula I, a process for severely damaging or killing unwanted plants by applying to the plants or to the growth medium of the plants an effective amount of a compound of formula I, processes for the preparation of compounds of formula I and intermediates useful in the preparation of compounds of formula I.

EP 0 013 143 A2

0013143

TITLE

Herbicidal compounds and compositions and process for the use thereof

This invention relates to organic compounds having biological activity and in particular to organic compounds having herbicidal properties, to processes for the preparation of such compounds, to intermediates useful in the preparation of such compounds and to herbicidal compositions and processes utilizing such compounds.

We have now found a new class of pyrimidines which exhibit biological activity, and in particular herbicidal activity.

Accordingly the invention provides a compound of formula I:

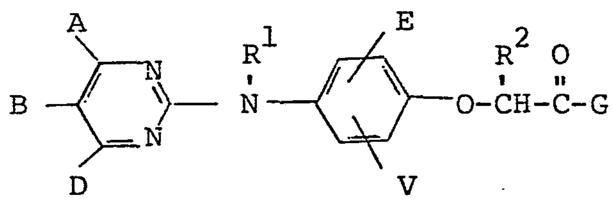

I

or a salt thereof wherein:

A, B, D, E and V are independently chosen from the group consisting of hydrogen, halogen, nitro, cyano, thiocyano, amino, $C_1$ to $C_6$ alkylamino, di($C_1$ to $C_6$ alkyl)amino, $C_1$ to $C_6$ alkyl, $C_1$ to $C_6$ haloalkyl, $C_2$ to $C_6$ alkenyl, $C_3$ to $C_7$ cycloalkyl, $C_1$ to $C_6$ alkoxy, $C_1$ to $C_6$ haloalkoxy, $C_1$ to $C_6$ alkylthio, $C_1$ to $C_6$ alkylsulfinyl, $C_1$ to $C_6$ alkylsulfonyl, $C_1$ to $C_6$ haloalkylsulfinyl, $C_1$ to $C_6$ haloalkylsulfonyl, sulfo, $C_1$ to $C_6$ alkoxysulfonyl, sulfamoyl, N-($C_1$ to $C_6$ alkyl)sulfamoyl, N,N-di($C_1$ to $C_6$ alkyl)sulfamoyl, carboxy, ($C_1$ to $C_6$ alkoxy)-carbonyl, carbamoyl, N-($C_1$ to $C_6$ alkyl)carbamoyl, N,N-di($C_1$ to $C_6$ alkyl)carbamoyl, phenyl, phenoxy, phenylthio, and the groups substituted phenyl, substituted phenoxy and substituted phenylthio wherein in each group the phenyl ring is substituted with one to three

substituents chosen from the group consisting of halogen, $C_1$ to $C_6$ alkyl, $C_1$ to $C_6$ haloalkyl, $C_1$ to $C_6$ alkoxy, nitro and cyano;

$R^1$ is chosen from the group consisting of hydrogen, $C_1$ to $C_{10}$ alkyl, $C_2$ to $C_{10}$ alkenyl, $C_2$ to $C_{10}$ alkoxyalkyl, $C_1$ to $C_{10}$ haloalkyl, formyl, $C_2$ to $C_{10}$ alkanoyl, phenyl, benzyl, benzoyl, and the groups phenyl, benzyl and benzoyl wherein in each group the phenyl ring is substituted with one or two substituents chosen from the group consisting of halogen, $C_1$ to $C_6$ alkyl, $C_1$ to $C_6$ haloalkyl, $C_1$ to $C_6$ alkoxy, nitro and cyano;

$R^2$ is chosen from the group consisting of hydrogen, $C_1$ to $C_6$ alkyl, $C_2$ to $C_6$ alkenyl, $C_2$ to $C_6$ alkoxyalkyl, $C_1$ to $C_6$ haloalkyl, acetyl, propionyl, and $C_2$ to $C_6$ alkoxycarbonyl; and

G is chosen from the group consisting of hydroxy, mercapto, $C_1$ to $C_{10}$ alkoxy, $C_1$ to $C_{10}$ haloalkoxy, $C_2$ to $C_{10}$ alkenyloxy, $C_2$ to $C_{10}$ alkynyloxy, $C_1$ to $C_{10}$ alkylthio, $C_2$ to $C_{10}$ alkenylthio, $C_2$ to $C_{10}$ alkynylthio, $C_3$ to $C_7$ cycloalkoxy, $C_3$ to $C_7$ cycloalkoxy substituted with one or two $C_1$ to $C_4$ alkyl groups, phenoxy, phenylthio, benzyloxy, benzylthio, the group $C_1$ to $C_{10}$ alkoxy substituted with a substituent chosen from the group consisting of $C_1$ to $C_6$ alkoxy, amino, ammonio, cyano, N-($C_1$ to $C_6$ alkyl)amino, N,N-di($C_1$ to $C_6$ alkyl)-amino and N,N,N-tri($C_1$ to $C_6$ alkyl)ammonio, the groups phenoxy, phenylthio, benzyloxy and benzylthio wherein in each group the phenyl ring is substituted with one or two substituents chosen from the group consisting of halogen, nitro, cyano, $C_1$ to $C_6$ alkyl, $C_1$ to $C_6$ haloalkyl and $C_1$ to $C_6$ alkoxy, the group OM wherein M is the cation of an inorganic or organic base, the group $-NHSO_2R^3$ wherein $R^3$ is chosen from $C_1$ to $C_{10}$ alkyl and $C_1$ to $C_{10}$ haloalkyl, and the group $-NR^4R^5$

wherein $R^4$ and $R^5$ are independently chosen from the group consisting of hydrogen, $C_1$ to $C_6$ alkyl, phenyl and benzyl or $R^4$ and $R^5$ together form a heterocyclic ring.

Suitable A, B, D, E and V include hydrogen, halogen, nitro, cyano, thiocyano, amino, $C_1$ to $C_6$ alkylamino, di($C_1$ to $C_6$ alkyl)amino, $C_1$ to $C_6$ alkyl, $C_1$ to $C_6$ haloalkyl, $C_2$ to $C_6$ alkenyl, $C_3$ to $C_7$ cycloalkyl, $C_1$ to $C_6$ alkoxy, $C_1$ to $C_6$ alkylthio, ($C_1$ to $C_6$ alkoxy)-carbonyl, phenyl, phenoxy, phenylthio and the groups substituted phenyl, substituted phenoxy and substituted phenylthio wherein in each group the phenyl ring is substituted with one to three substituents chosen from the group consisting of halogen, $C_1$ to $C_6$ alkyl, $C_1$ to $C_6$ haloalkyl, $C_1$ to $C_6$ alkoxy, nitro and cyano.

Suitable $R^1$ include hydrogen, $C_1$ to $C_{10}$ alkyl, $C_2$ to $C_{10}$ alkenyl, $C_2$ to $C_{10}$ alkoxyalkyl, $C_1$ to $C_{10}$ haloalkyl, formyl, $C_2$ to $C_{10}$ alkanoyl, phenyl, benzyl, benzoyl and the groups phenyl, benzyl and benzoyl wherein in each group the phenyl ring is substituted with one or two substituents chosen from the group consisting of halogen, $C_1$ to $C_6$ alkyl, $C_1$ to $C_6$ haloalkyl, $C_1$ to $C_6$ alkoxy, nitro and cyano.

Suitable $R^2$ include hydrogen, $C_1$ to $C_6$ alkyl, $C_2$ to $C_6$ alkenyl, $C_2$ to $C_6$ alkoxyalkyl, $C_1$ to $C_6$ haloalkyl, acetyl, propionyl and $C_2$ to $C_6$ alkoxycarbonyl.

Suitable G include hydroxy, mercapto, $C_1$ to $C_{10}$ alkoxy, $C_1$ to $C_{10}$ haloalkoxy, $C_2$ to $C_{10}$ alkenyloxy, $C_2$ to $C_{10}$ alkynyloxy, $C_1$ to $C_{10}$ alkylthio, $C_2$ to $C_{10}$ alkenylthio, $C_3$ to $C_7$ cycloalkoxy, $C_3$ to $C_7$ cycloalkoxy substituted with one or two $C_1$ to $C_4$ alkyl groups, phenoxy, phenylthio, benzyloxy, benzylthio, the group $C_1$ to $C_{10}$ alkoxy substituted with a $C_1$ to $C_6$ alkoxy group, the groups phenoxy, phenylthio, benzyloxy and benzylthio wherein in each group the phenyl ring is substituted with one or two substituents chosen from the group consisting of halogen, nitro, cyano, $C_1$ to $C_6$ alkyl, $C_1$ to $C_6$ haloalkyl and $C_1$ to $C_6$ alkoxy, the group OM wherein M is the

cation of an inorganic or organic base, the group $-NHSO_2R^3$ wherein $R^3$ is chosen from $C_1$ to $C_{10}$ alkyl and $C_1$ to $C_{10}$ haloalkyl, and the group $-NR^4R^5$ wherein $R^4$ and $R^5$ are independently chosen from the group consisting of hydrogen, $C_1$ to $C_6$ alkyl, phenyl and benzyl or $R^4$ and $R^5$ together form a heterocyclic ring.

When G is the group OM suitable M include alkali metal ions, alkaline earth metal ions and the ammonium ion $H\overset{\oplus}{N}R^6R^7R^8$ wherein $R^6$, $R^7$ and $R^8$ are independently chosen from hydrogen, $C_1$ to $C_6$ alkyl, $C_2$ to $C_6$ hydroxyalkyl, phenyl and benzyl.

When G is the group $-NR^4R^5$ wherein $R^4$ and $R^5$ together form a heterocyclic ring suitable heterocyclic rings include morpholino, piperidino, 1-piperazinyl and 1-pyrrolidinyl.

Preferred A, B and D include hydrogen, halogen, nitro, cyano, phenyl, $C_1$ to $C_6$ alkyl and $C_1$ to $C_6$ haloalkyl.

Preferred E and V include hydrogen and halogen.

Preferred $R^1$ include hydrogen, $C_1$ to $C_6$ alkyl, $C_1$ to $C_6$ alkanoyl and benzyl.

Preferred $R^2$ include hydrogen and $C_1$ to $C_6$ alkyl.

Preferred G include hydroxy, $C_1$ to $C_{10}$ alkoxy, $C_2$ to $C_{10}$ alkenyloxy, $C_2$ to $C_{10}$ alkynyloxy, $C_3$ to $C_7$ cycloalkoxy, benzyloxy, $C_1$ to $C_{10}$ alkoxy substitued with a substituent chosen from amino, ammonio, N-($C_1$ to $C_6$ alkyl)amino, N,N-di($C_1$ to $C_6$ alkyl)amino and N,N,N-tri($C_1$ to $C_6$ alkyl)ammonio, and the group OM wherein M is an alkali metal or alkaline earth metal ion.

More preferred A, D, E and V are hydrogen.

More preferred B include chlorine, bromine, iodine, nitro, phenyl and trifluoromethyl.

More preferred $R^1$ include hydrogen, methyl and ethyl.

More preferred $R^2$ is methyl.

More preferred G include hydroxy, $C_1$ to $C_6$ alkoxy, $C_2$ to $C_6$ alkenyloxy, $C_2$ to $C_6$ alkynyloxy and $C_1$ to $C_6$ alkoxy substituted with a group chosen from N,N-di($C_1$ to $C_6$ alkyl)amino and N,N,N-tri($C_1$ to $C_6$ alkyl)ammonio.

Examples of the compounds embraced by the invention include:

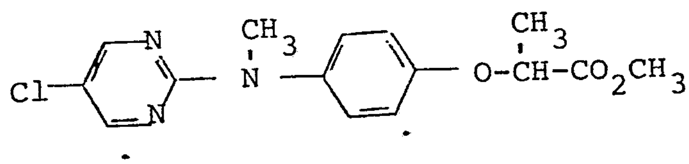

2

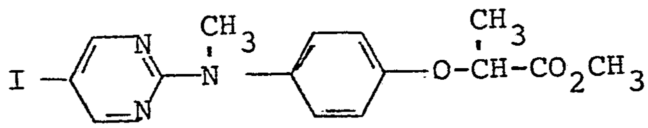

3

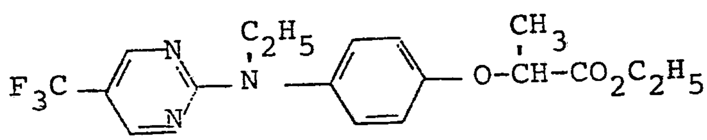

22

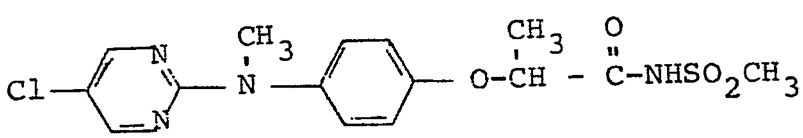

23

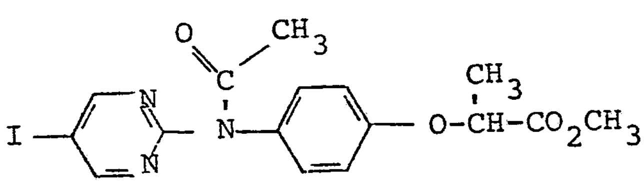

24

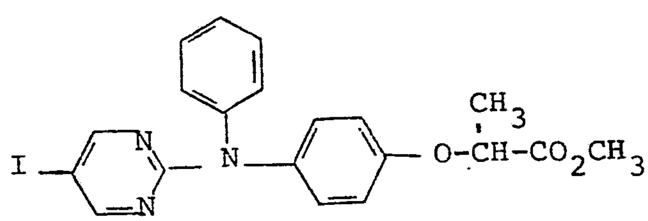

25

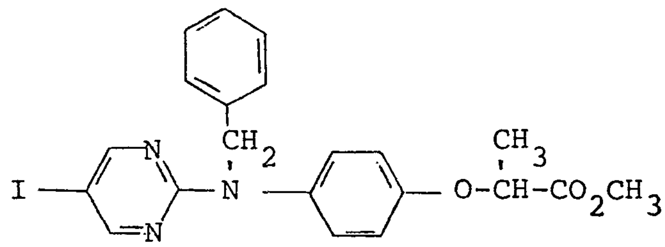

26

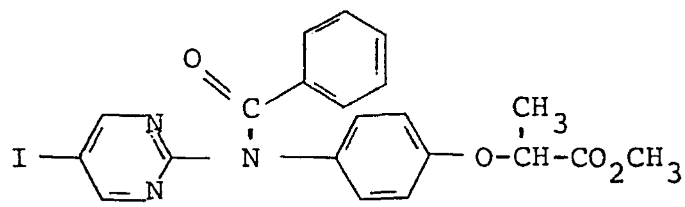

27

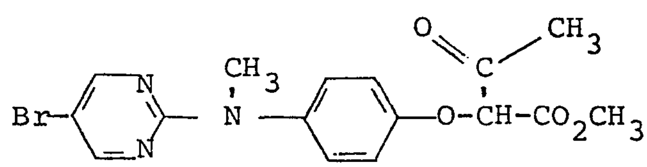

28

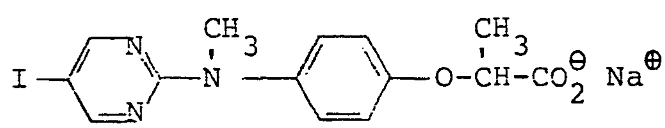

29

The compounds of formula I wherein $R^2$ is not hydrogen are optically active and the present invention also includes the individual stereo isomers of such compounds and mixtures of those stereo isomers, in addition to the racemic mixture of stereo isomers.

Specific examples of the compounds of the invention are detailed in Table 1 below.

TABLE 1

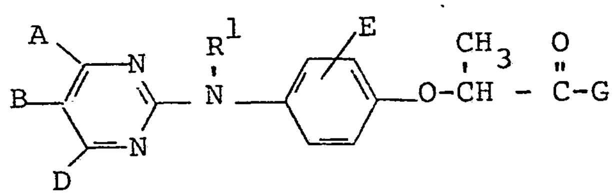

| Compound No | Substituents | | | | | |
|---|---|---|---|---|---|---|
| | A | B | D | E | $R^1$ | G |
| 1 | H | Br | H | H | H | $OCH_3$ |
| 2 | H | Cl | H | H | $CH_3$ | $OCH_3$ |
| 3 | H | I | H | H | $CH_3$ | $OCH_3$ |
| 4 | H | Cl | H | H | $2\text{-}C_3H_7$ | $OCH_3$ |
| 5 | H | Cl | H | H | H | $OCH_3$ |
| 6 | H | Cl | H | H | $CH_3\overset{\mathrm{O}}{\overset{\|}{C}}$ | $OCH_3$ |
| 7 | H | $CF_3$ | H | H | $CH_3$ | $OCH_3$ |
| 8 | $CH_3$ | H | $CH_3$ | H | $CH_3$ | $OCH_3$ |
| 9 | H | $CF_3$ | H | H | $C_2H_5$ | $OCH_3$ |
| 10 | H | $CH_3$ | H | H | $CH_3$ | $OCH_3$ |
| 11 | H | Br | H | H | $CH_3$ | $OCH_3$ |
| 12 | H | Br | H | H | $C_2H_5$ | $OCH_3$ |
| 13 | H | Br | H | 3-Cl | H | $OCH_3$ |
| 14 | H | H | H | H | $CH_3$ | $OCH_3$ |
| 15 | H | $NO_2$ | H | H | $CH_3$ | $OCH_3$ |
| 16 | H | Br | H | H | H | OH |
| 17 | H | Ph | H | H | $CH_3$ | $OCH_3$ |
| 18 | H | Br | H | H | $CH_3$ | $OC_4H_7\text{-}n$ |
| 19 | H | Br | H | H | $1\text{-}C_3H_7$ | OH |
| 20 | H | Br | H | H | $CH_3$ | $OCH_2CH_2N(CH_3)_2$ |
| 21 | H | Br | H | H | $CH_3$ | $OCH_2CH_2\overset{\oplus}{N}(CH_3)_3\overset{\ominus}{I}$ |

The compounds of the invention may be prepared by a variety of methods and in a further aspect the invention provides methods for the preparation of the compounds of formula I.

Compounds of formula I wherein G is not hydroxy may be prepared from the corresponding acid of formula Ia (I; G=OH) by any of the conventional methods known in the art for the conversion of a carboxylic acid to an acid salt, acid ester, acid amide or acid hydrazide (SCHEME A).

SCHEME A

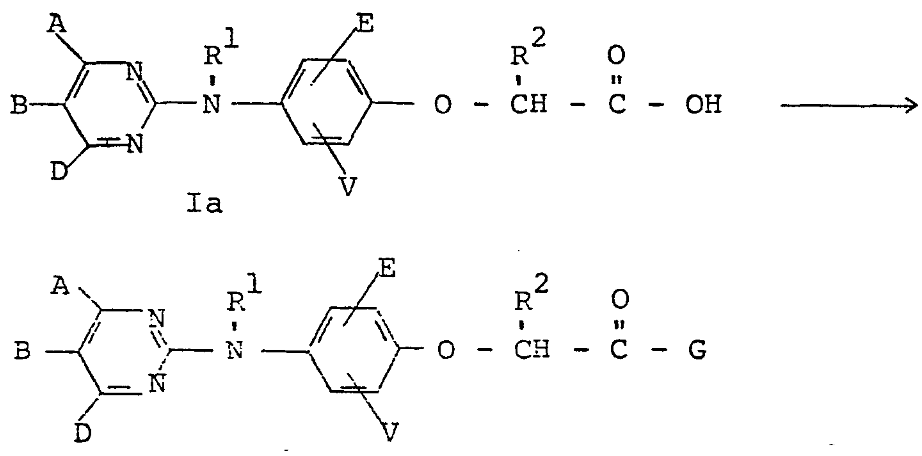

Ia

Compounds of formula I wherein $R^1$ is not hydrogen may be prepared from the corresponding secondary amine Ib (I; $R^1$=H) by any of the conventional methods known in the art for alkylation or acylation of secondary amines (SCHEME B).

SCHEME B

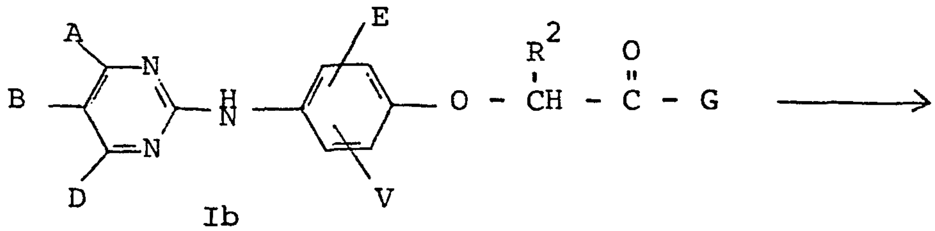

Ib

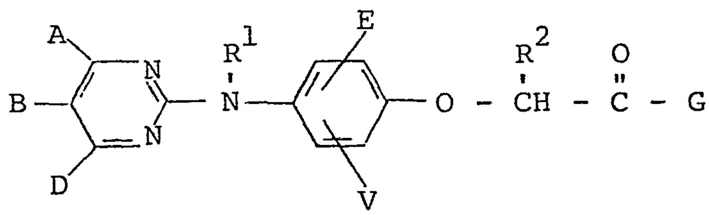

I

Compounds of formula I wherein A, B, D, E, V, $R^1$, $R^2$ and G are as hereinbefore defined may be prepared by the condensation of a phenol of II with a compound of formula III wherein hal is chlorine, bromine or iodine preferably in the presence of an alkaline material. (SCHEME C).

SCHEME C

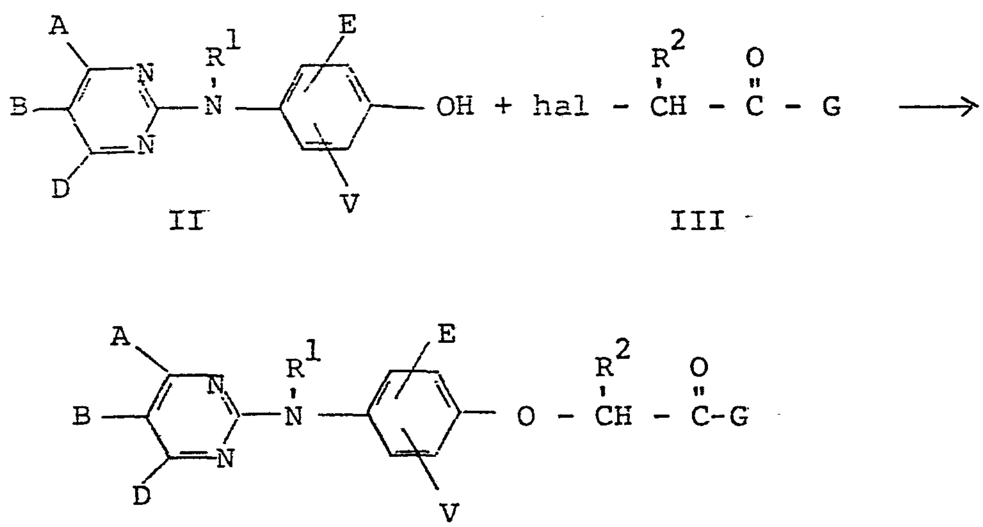

II

III

I

Alternatively, compounds of formula I wherein A, B, D, E, V, $R^1$, $R^2$ and G are as hereinbefore defined may be prepared by :

a) the condensation of the appropriate pyrimidine of formula IV, wherein L is a leaving group (for

example, alkylsulfonyl, chlorine, bromine or iodine) with the appropriate aniline of formula V (SCHEME D).

SCHEME D

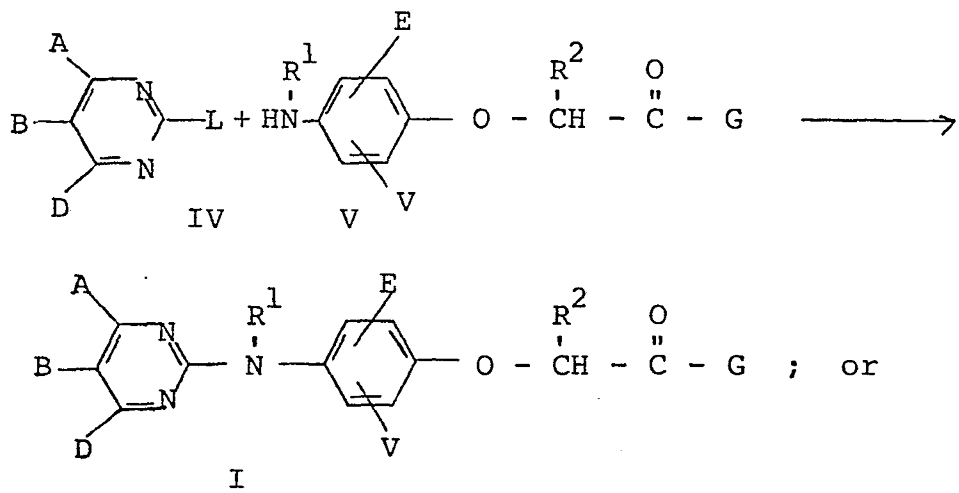

IV V

I

; or

b) the following steps in sequence:

(i) the condensation of the appropriate pyrimidine of formula IV, wherein L is a leaving group (for example, alkylsulfonyl, chlorine, bromine or iodine) with the appropriate aniline of formula VI, wherein Q is hydroxy or $C_1$ to $C_6$ alkoxy to give a compound of formula VII wherein Q is hydroxy or $C_1$ to $C_6$ alkoxy;

(ii) the dealkylation of the compound of formula VII, wherein Q is $C_1$ to $C_6$ alkoxy prepared in step (i) above to give a phenol of formula II; and

(iii) the condensation of the phenol of formula II prepared as described in step (i) or step (ii) above with a compound of formula III according to the process described for SCHEME C above. (Steps (i) and (ii) are shown in SCHEME E).

SCHEME E

(i)

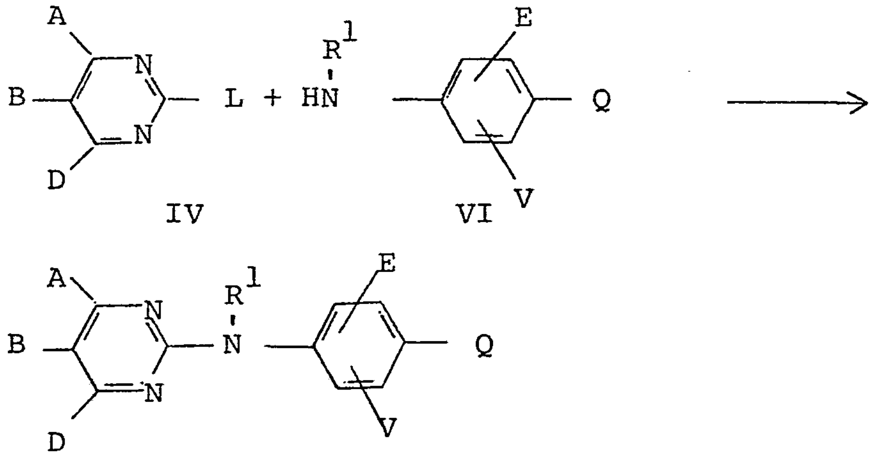

IV VI

VII

(ii)

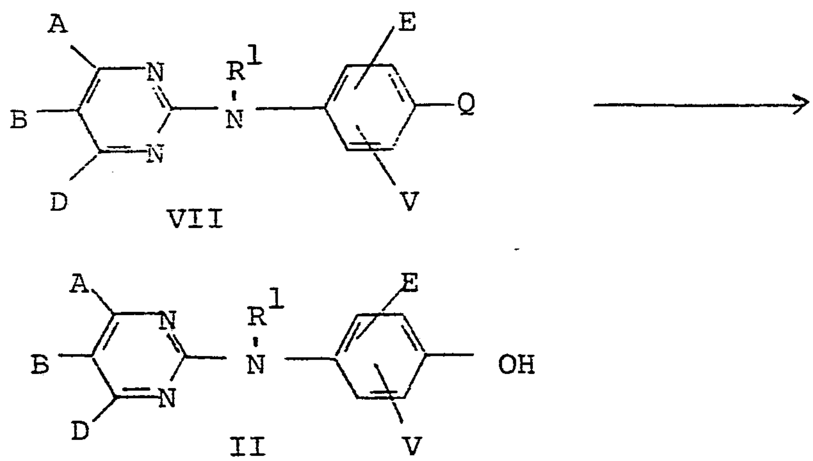

VII

II

In a further process, compounds of formula I wherein A, B, D, E, V, $R^1$, $R^2$ and G are as hereinbefore defined may be prepared by the following steps in sequence.

(i) the reaction of cyanamide with the appropriate aniline of formula VI, wherein Q is hydroxy or $C_1$ to $C_6$ alkoxy, to give the guanidine of formula VIII;

(ii) the reaction of the guanidine of formula VIII prepared in step (i) above with an acrolein of formula IX, wherein T is a leaving group (for example, alkoxy or dimethylamino) to give the N-(2-pyrimidyl)-N-phenylamine of formula X;

(iii) the dealkylation of the compound of formula X prepared in step (ii) above, wherein Q is $C_1$ to $C_6$ alkoxy, to give a product of formula XI according to the process described in SCHEME E step (ii) for the dealkylation of a compound of formula VII; and

(iv) the condensation of the product of formula XI prepared in step (ii) or step (iii) above with a compound of formula III according to the process described in SCHEME C above for the reaction of a compound of formula II with a compound of formula III (Steps (i), (ii) and (iii) are shown in SCHEME F).

SCHEME F

(i)

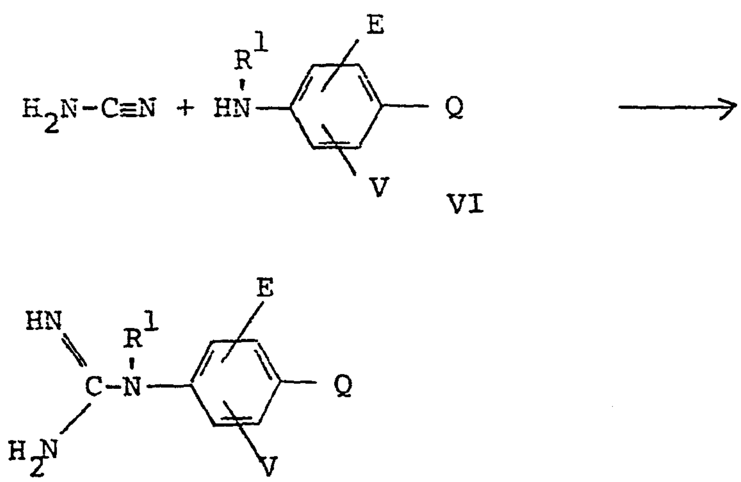

VI

VIII

(ii)

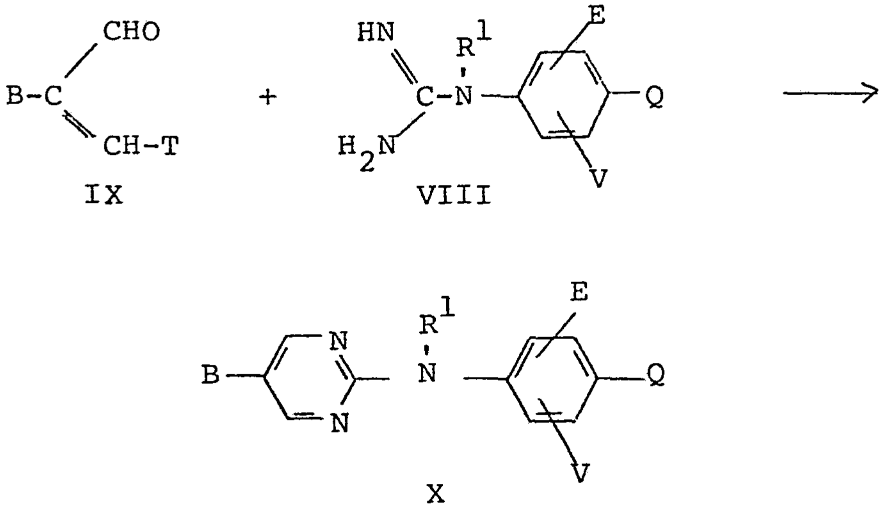

IX VIII

X

(iii)

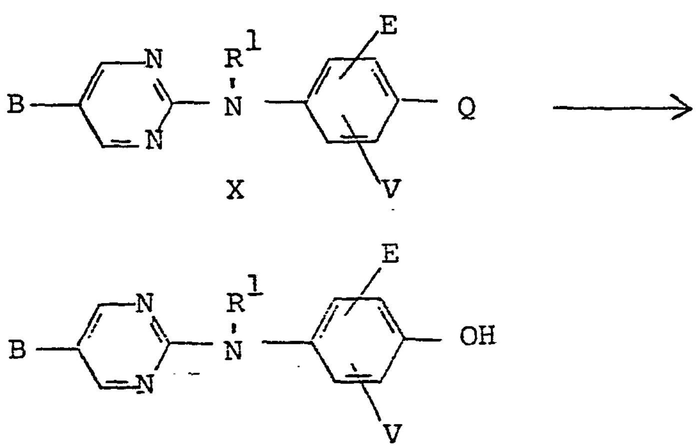

X

XI

The condensation reaction illustrated in SCHEME C and outlined above is preferably carried out in the presence of an alkaline material and preferably in the presence of a solvent. Suitable alkaline materials include, for example, the alkali and alkaline earth metal hydroxides and carbonates such as sodium hydroxide, potassium hydroxide, sodium carbonate and potassium carbonate. Suitable solvents include ketones such as, for example, acetone, methyl ethyl ketone and methyl isobutyl ketone, and dipolar aprotic solvents such as, for example, dimethylformamide, dimethylacetamide,

dimethylsulfoxide, N-methylpyrrolidone, hexamethylphosphoramide and sulfolan.

The condensation reactions illustrated in SCHEMES D and E step (i) and outlined above are preferably carried out in the presence of a solvent.

The reaction conditions required to effect the condensation reactions illustrated in SCHEMES C, D and E step (i) and outlined above vary according to the nature of the reactants and the solvent used. In general the reaction is facilitated by the application of heat and usually a reaction temperature in the range of 40° to 150°C and reaction time of between 0.5 and 20 hours is satisfactory. However, higher or lower reaction temperatures and/or shorter or longer reaction times may be used if desired.

The dealkylation reactions illustrated in SCHEMES E step (ii) and F step (iii) outlined above may be effected using a variety of reagents known in the art. For example, aryl-alkyl ethers may be cleaved using reagents such as pyridine hydrochloride, hydriodic acid, hydrobromic acid, sodium thioethoxide in dimethylformamide, acetyl p-toluene-sulphonate, sodium or potassium iodide in formic or acetic acid, lithium iodide in 2,4,6-collidine and boron tribromide. Reaction times and reaction conditions vary widely depending on the dealkylation agent used and the ether to be cleaved. The reaction conditions generally employed when using the above "ether-cleavage" reagents are known to those skilled in the art and may be adapted without undue experimentation to effect the "ether-cleavage" reactions illustrated in SCHEMES E step (ii) and F step (iii) outlined above.

The compounds of formula VII

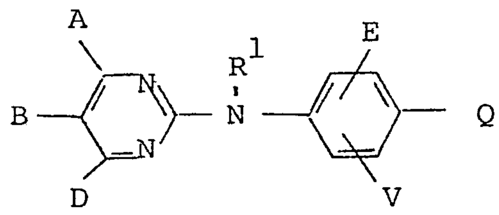

VII,

which are useful intermediates in the preparation of compounds of formula I, are novel compounds. Therefore, in a further embodiment the invention provides compounds of formula VII wherein A, B, D, E, V, $R^1$ and Q are as hereinbefore defined.

The compounds of formula I are active as herbicides and therefore, in a further aspect the invention provides a process for severely damaging or killing unwanted plants which process comprises applying to the plants, or to the growth medium of the plants, an effective amount of a compound of formula I as hereinbefore defined.

Generally speaking the compounds of formula I are herbicidally effective against a variety of plants. However, certain of the compounds of the invention are selectively active against monocotyledonous plants, dicotyledonous plants being relatively unaffected by rates of application of the compunds of the invention which are severely damaging or lethal to other plant species. Therefore, in yet a further aspect the invention provides a process for selectively controlling the growth of weeds in crops which process comprises applying to the crop, or to the growth medium of the crop, a compound of formula I, as hereinbefore defined, in an amount sufficient to severely damage or kill the weeds but insufficient to damage the crop substantially.

The compounds of formula I may be applied directly to the plant (post-emergence application) or to the soil before the emergence of the plant (pre-emergence application).

The compounds of formula I may be used on their own to inhibit the growth of, severely damage, or kill plants but are preferably used in the form of a composition comprising a compound of the invention in admixture with a carrier comprising a solid or liquid diluent. Therefore, in yet a further aspect the invention provides plant growth inhibiting, plant damaging, or plant killing compositions comprising a compound of formula I as hereinbefore defined and an inert carrier therefor.

Compositions according to the invention include both dilute compositions, which are ready for immediate use, and concentrated compositions, which require to be diluted before use, usually with water. Preferably the compositions contain from 0.01% to 90% by weight of the active ingredient. Dilute compositions ready for use preferably contain from 0.01 to 2% of active ingredient, while concentrated compositions may contain from 20 to 90% of active ingredient, although from 20 to 70% is usually preferred.

The solid compositions may be in the form of granules, or dusting powders wherein the active ingredient is mixed with a finely divided solid diluent, eg kaolin, bentonite, kieselguhr, dolomite, calcium carbonate, talc, powdered magnesia, Fuller's earth and gypsum. They may also be in the form of dispersible powders or grains, comprising a wetting agent to facilitate the dispersion of the powder or grains in liquid. Solid compositions in the form of a powder may be applied as foliar dusts.

Liquid compositions may comprise a solution or dispersion of an active ingredient in water optionally containing a surface-active agent, or may comprise a solution or dispersion of an active ingredient in a water-immiscible organic solvent which is dispersed as

droplets in water.

Surface-active agents may be of the cationic, anionic, or non-ionic type. The cationic agents are, for example, quaternary ammonium compounds (eg cetyltrimethylammonium bromide). Suitable anionic agents are soaps; salts of aliphatic mono esters of sulphuric acid, for example sodium lauryl sulphate; and salts of sulphonated aromatic compounds, for example sodium dodecylbenzenesulphonate, sodium, calcium, and ammonium lignosulphonate, butylnaphthalene sulphonate, and a mixture of the sodium salts of diisopropyl- and triisopropylnaphthalenesulphonic acid. Suitable non-ionic agents are the condensation products of ethylene oxide with fatty alcohols such as oleyl alcohol and cetyl alcohol, or with alkylphenols such as octyl- or nonylphenol or octylcresol. Other non-ionic agents are the partial esters derived from long chain fatty acids and hexitol anhydrides, for example sorbitan monolaurate; the condensation products of the partial ester with ethylene oxide; and the lecithins.

The aqueous solutions or dispersions may be prepared by dissolving the active ingredient in water or an organic solvent optionally containing wetting or dispersing agent(s) and then, when organic solvents are used, adding the mixtures so obtained to water optionally containing wetting or dispersing agent(s). Suitable organic solvents include, for example, ethylene dichloride, isopropyl alcohol, propylene glycol, diacetone alcohol, toluene, kerosene, methylnapthalene, the xylenes trichloroethylene.

The compositions for use in the form of aqueous solutions or dispersions are generally supplied in the form of a concentrate containing a high proportion of the active ingredient, and the concentrate is then diluted with water before use. These concentrates are usually

required to withstand storage for prolonged periods and after such storage, to be capable of dilution with water to form aqueous preparations which remain homogeneous for a sufficient time to enable them to be applied by conventional spray equipment. Concentrates conveniently contain 20-90%, preferably 20-70%, by weight of the active ingredient(s). Dilute preparations ready for use may contain varying amounts of the active ingredient(s) depending upon the intended purpose; amounts of 0.01% to 10.0% and preferably 0.1% to 2%, by weight of active ingredient(s) are normally used.

A preferred form of concentrated composition comprises the active ingredient which has been finely divided and which has been dispersed in water in the presence of a surface-active agent and a suspending agent. Suitable suspending agents are hydrophilic colloids and include, for example, polyvinylpyrrolidone and sodium carboxymethylcellulose, and the vegetable gums, for example gum acacia and gum tragacanth. Preferred suspending agents are those which impart thixotropic properties to, and increase the viscosity of the concentrate. Examples of preferred suspending agents include hydrated colloidal mineral silicates, such as montmorillonite, beidellite, nontronite, hectorite, saponite, and saucorite. Bentonite is especially preferred. Other suspending agents include cellulose derivatives and polyvinyl alcohol.

The rate of application of the compounds of the invention will depend on a number of factors including, for example, the compound chosen for use, the identity of the plants whose growth is to be inhibited the formulation selected for use and whether the compound is to be applied for foliage or root uptake. As a general guide, however, an application rate of from 0.05 to 20 kilograms per hectare is suitable while from 0.1 to 10

kilograms per hectare may be preferred.

The compositions of the invention may comprise, in addition to one or more compounds of the invention, one or more compounds not of the invention but which possess biological activity. For example, as hereinbefore indicated the compounds of the invention are in general substantially more effective against monocotyledonous plants or grass species than against dicotyledonous plants or broad-leaved species. As a result, in certain applications the herbicidal use of the compounds of the invention alone may be insufficient to protect a crop. Accordingly in yet a still further embodiment the invention provides a herbicidal composition comprising a mixture of at least one herbicidal compound of formula I as hereinbefore defined with at least one other herbicide.

The other herbicide may be any herbicide not having the formula I. It will generally be a herbicide having a complementary action. For example, one preferred class is of mixtures comprising a herbicide active against broad-leaved weeds. A second preferred class is of mixtures comprising a contact herbicide.

Examples of useful complementary herbicides include:

A. benzo-2,1,3-thiadiazin-4-one-2,2-dioxides such as 3-isopropylbenzo-2,1,3-thiadiazin-4-one-2,2-dioxide (common name bentazon);

B. hormone herbicides and in particular the phenoxyalkanoic acids such as 4-chloro-2-methylphenoxyacetic acid (common name MCPA), 2-(2,4-dichlorophenoxy)propionic acid (common name dichlorprop), 2,4,5-trichlorophenoxyacetic acid (common name 2,4,5-T), 4-(4-chloro-2-methylphenoxy)butyric acid (common name MCPB), 2,4-dichlorophenoxyacetic acid (common name 2,4-D), 4-(2,4-dichlorophenoxy)butyric

acid (common name 2,4-DB), 2-(4-chloro-2-methylphenoxy)propionic acid (common name mecoprop), and their derivatives (eg salts, esters, amides and the like);

C. 3-/4-(4-halophenoxy)phenyl7-1,1-dialkylureas such as 3-/4-(4-chlorophenoxy)phenyl7-1,1-dimethylurea (common name chloroxuron);

D. dinitrophenols and their derivatives (eg acetates) such as 2-methyl-4,6-dinitrophenol (common name DNOC), 2-tertiarybutyl-4,6-dinitrophenol (common name dinoterb), 2-secondarybutyl-4,6-dinitrophenol (common name dinoseb) and its ester dinoseb acetate;

E. dinitroaniline herbicides such as N',N'-diethyl-2,6-dinitro-4-trifluoromethyl-m-phenylenediamine (common name dinitramine), 2,6-dinitro-N,N-dipropyl-4-trifluoromethylaniline (common name trifluralin) and 4-methylsulfonyl-2,6-dinitro-N,N-dipropylaniline (common name nitralin);

F. phenylurea herbicides such as N'-(3,4-dichlorophenyl)-N,N-dimethylurea (common name diuron) and N,N-dimethyl-N'-/3-(trifluoromethyl)phenyl7urea (common name fluometuron);

G. phenylcarbamoyloxyphenylcarbamates such as 3-/(methoxycarbonyl)amino7phenyl (3-methylphenyl)-carbamate (common name phenmedipham) and 3-/(ethoxycarbonyl)amino7phenyl phenylcarbamate (common name desmedipham);

H. 2-phenylpyridazin-3-ones such as 5-amino-4-chloro-2-phenylpyridazin-3-one (common name pyrazon);

I. uracil herbicides such as 3-cyclohexyl-5,6-trimethyleneuracil (common name lenacil), 5-bromo-3-sec-butyl-6-methyluracil (common name bromacil) and 3-tert-butyl-5-chloro-6-methyluracil (common

name terbacil);

J. triazine herbicides such as 2-chloro-4-ethylamino-6-(iso-propylamino)-1,3,5-triazine (common name atrazine), 2-chloro-4,6-di(ethylamino)-1,3,5-triazine (common name simazine) and 2-azido-4-(iso-propylamino)-6-methylthio-1,3,5-triazine (common name aziprotryne);

K. 1-alkoxy-1-alkyl-3-phenylurea herbicides such as 3-(3,4-dichlorophenyl)-1-methoxy-1-methylurea (common name linuron), 3-(4-chlorophenyl)-1-methoxy-1-methylurea (common name monolinuron) and 3-(4-bromo-4-chlorophenyl)-1-methoxy-1-methylurea (common name chlorobromuron);

L. thiolcarbamate herbicides such as S-propyl dipropylthiocarbamate (common name vernolate);

M. 1,2,4-triazin-5-one herbicides such as 4-amino-4,5-dihydro-3-methyl-6-phenyl-1,2,4-triazine-5-one (common name metamitron) and 4-amino-6-tert-butyl-4,5-dihydro-3-methylthio-1,3,4-triazin-5-one (common name metribuzin);

N. benzoic acid herbicides such as 2,3,6-trichlorobenzoic acid (common name 2,3,6-TBA), 3,6-dichloro-2-methoxybenzoic acid (common name dicamba) and 3-amino-2,5-dichlorobenzoic acid (common name chloramben).

O. anilide herbicides such as N-butoxymethyl-α-chloro-2',6'-diethylacetanilide (common name butachlor)-the correspnding N-methoxy compound (common name alachlor), the corresponding N-iso-propyl compound (common name propachlor) and 3',4'-dichloropropionanilide (common name propanil);

P. dihalobenzonitrile herbicides such as 2,6-dichlorobenzonitrile (common name dichlobenil), 3,5-dibromo-4-hydroxybenzonitrile (common name bromoxynil) and 3,5-diiodo-4-hydroxybenzonitrile (common name ioxynil).

Q. haloalkanoic herbicides such as 2,2-dichloropropionic acid (common name dalapon), trichloroacetic acid (common name TCA) and salts thereof;

R. diphenylether herbicides such as 4-nitrophenyl 2-nitro-4-trifluoromethylphenyl ether (common name fluorodifen), methyl 5-(2,4-dichlorophenoxy)-2-nitrobenzoate (common name bifenox), 2-nitro-5-(2-chloro-4-trifluoromethylphenoxy)benzoic acid and 2-chloro-4-trifluoromethylphenyl 3-ethoxy-4-nitrophenyl ether; and

S. miscellaneous herbicides including N,N-dimethyldiphenylacetamide (common name diphenamid), N-(1-naphthyl)phthalamic acid (common name naptalam) and 3-amino-1,2,4-triazole.

Examples of useful contact herbicides include:

T. bipyridylium herbicides such as those in which the active entity is the 1,1'-dimethyl-4,4'-dipyridylium ion (common name paraquat) and those in which the active entity is the 1,1'-ethylene-2,2'-dipyridylium ion (common name diquat);

U. organoarsenical herbicides such as monosodium methanearsonate (common name MSMA); and

V. amino acid herbicides such as N-(phosphonomethyl)-glycine (common name glyphosate) and its salts and esters.

The invention is now illustrated by, but in no way limited to, the following Examples.

Example 1

Preparation of Methyl 2-/4-(5-Chloro-N-methyl-2-pyrimidylamino)phenoxy7propionate (2)

a) 1-Methyl-1-(4-hydroxyphenyl)guanidine sulfate. A mixture of 4-N-methylaminophenol sulfate (7 g), cyanamide (1.3 g) and ethanol (20 ml) was heated under under reflux for a period of 20 hr. The reaction mixture was cooled to a temperature of 0°C, held at that temperature for a period of 72 hr and then the guanidine salt was collected by filtration as a grey crystalline solid, mp 285°C (dec) /ref. J. Med. Chem., 18, 1077 (1975)7.

b) 4-(N-Methyl-5-chloro-2-pyrimidylamino)phenol. A mixture of 2-chloro-3-dimethylaminoacrolein (2.7 g) /ref. Z. Arnold, Coll. Czech. Chem. Comm., 26, 2852 (1961)7, anhydrous potassium carbonate (2.8 g), 1-methyl-1-(4-hydroxyphenyl)guanidine sulfate (4.3 g) and methanol (50 ml) was heated under reflux for a period of 18 hr. The reaction mixture was cooled, poured into cold 1M hydrochloric acid (100 ml) and extracted with chloroform (2 x 100 ml). The chloroform extracts were dried and evaporated to give the pyrimidine (2.3 g) as a pale brown solid, mp 135°C.

c) Methyl 2-/4-(5-chloro-N-methyl-2-pyrimidylamino)-phenoxy7propionate (2)

A mixture of methyl 2-bromopropionate (0.9 g), 4-(N-methyl-5-chloro-2-pyrimidylamino)phenol (1.2 g), anhydrous potassium carbonate (0.8 g) and acetone (20 ml) was heated under reflux for a period of 20 hr. The acetone was removed by distillation under reduced pressure and the residue was partitioned between chloroform and water. The chloroform extracts were dried and evaporated to give a brown oil. Chromatography over silica gel (8 g) with chloroform elution gave the title compound which formed colourless crystals (1.2 g), mp 74°C.

Example 2

Preparation of Methyl 2-/4-(5-Iodo-N-methyl-2-pyrimidylamino)phenoxy/propionate (3)

a) 2-Iodo-3-ethoxyacrolein.

Sodium hydride (0.5 g) was added with stirring to a solution of iodomalonaldehyde (4.0 g) in dimethylformamide (20 ml) held at a temperature of 20°C. After the evolution of hydrogen had ceased, ethyl iodide (4.0 g) was added to the solution and the mixture was stirred at a temperature of 20°C for a period of 24 hr. The dimethylformamide was removed by distillation under reduced pressure at a temperature below 40°C and the residue was partitioned between chloroform and water. The chloroform extracts were dried and the chloroform was removed by distillation under reduced pressure to give the acrolein as a brown oil. The product was characterised by its p.m.r. spectrum (chemical shift δ in ppm): 1.5, t(3H); 4.5, q(2H); 7.8, s(1H); and 8.9, s(1H).

b) 4-(N-Methyl-5-iodo-2-pyrimidylamino)phenol.

A mixture of 2-iodo-3-ethoxyacrolein (2.4 g), anhydrous potassium carbonate (2.5 g), 1-methyl-1-(4-hydroxyphenyl)guanidine sulfate (3.0 g) and methanol (20 ml) was heated under reflux for a period of 2 hr. The reaction mixture was poured into ice cold 1M hydrochloric acid (100 ml) and extracted with chloroform. The chloroform extracts were dried and the chloroform was removed by distillation under reduced pressure to give the pyrimidine as a

brown oil (2.0 g). The product was characterised by its p.m.r. spectrum (chemical shift δ in ppm): 3.5, s(3H); 7.0, d of d(4H); 8.5, s(2H).

c) Methyl 2-/4-(5-iodo-N-methyl-2-pyrimidylamino-phenoxy/propionate (3)
The ester was prepared from 4-(N-methyl-5-iodo-2-pyrimidylamino)phenol and methyl 2-bromopropionate following the procedure described in Example 1 c) for the preparation of the analogous chloro-compound. The product, a colourless oil, was characterised by its p.m.r. spectrum (chemical shift δ in ppm): 1.6, d(3H); 3.4, s(3H); 4.8, q(1H); 7.2, d of d(4H); 8.5, s(2H).

Example 3

Preparation of Methyl 2-/4-(5-bromo-2-pyrimidylamino) phenoxy/propionate (1)

a) A mixture of p-anisidine (2.2 g), 5-bromo-2-chloropyrimidine (3.0 g), concentrated hydrochloric acid (0.3 ml), acetone (5 ml) and water (15 ml) was heated under reflux for a period of 5 hr.
The solution was cooled and the solid which precipitated was collected by filtration and characterised as 5-bromo-2-(4-methoxyphenylamino)pyrimidine (2.2 g), mp 132°C).

b) A solution of 5-bromo-2-(4-methoxyphenylamino) pyrimidine (2.2 g) in dichloromethane (20 ml) was cooled to a temperature of -89°C and boron tribromide (2 ml) was added to the stirred solution. The solution was allowed to warm to a temperature of 20°C and was then stirred for a period of 12 hr. Water (50 ml) was added to the reaction mixture which was then made alkaline with sodium hydrogen carbonate. The precipitated product 4-(5-bromo-2-pyrimidylamino)phenol (0.9 g) was collected by filtration, mp 182°C.

c) Methyl 2-[4-(5-bromo-2-pyrimidylamino)phenoxy]propionate was prepared from 4-(5-bromo-2-pyrimidylamino)phenol and methyl 2-bromopropionate following essentially the same procedure as that described in Example 1 part c). The product was a pale yellow solid, mp 110°C.

Example 4

Methyl 2-[4-(5-chloro-2-pyrimidylamino)phenoxy]propionate (5) was prepared from p-anisidine, 2,5-dichloropyrimidine and methyl 2-bromopropionate following essentially the same procedure as that described in Example 3 for the preparation of the bromo-analogue. The product was a colourless solid, mp 119°C.

Example 5

Methyl 2-[4-(5-chloro-N-isopropyl-2-pyrimidylamino)-phenoxy]propionate (4) was prepared from 1-isopropyl-1-(4-hydroxyphenyl) guanidine hydrochloride, 2-chloro-3-dimethylaminoacrolein and methyl 2-bromopropionate following essentially the same procedure as that described in Example 1 for the preparation of the N-methyl analogue. The product, an oil, was characterised by its p.m.r. spectrum (chemical shift δ in ppm): 1.1, d(6H); 1.6, d(3H); 3.8, s(3H); 4.6-5.3, m(2H); 6.9-7.2, m(4H); 8.3, s(2H).

Example 6

Preparation of Methyl 2-[4-(5-chloro-N-acetyl-2-pyrimidylamino)phenoxy]propionate (6)

a) A mixture of 5-chloro-2-(4-methoxyphenylamino)-pyrimidine (5.4 g, see Example 4), acetic anhydride (16 ml) and acetic acid (80 ml) was heated under reflux for a period of 24 hr. The mixture was concentrated by distillation under reduced pressure and the concentrate was poured onto crushed ice. The aqueous mixture was extracted with dichloromethane and the combined extracts were dried over anhydrous magnesium sulfate. After drying, the solvent was removed from the extracts by evaporation to give a brown solid. The residue crystallised from ethanol to give 5-chloro-2-(N-acetyl-4-methoxyphenylamino)pyrimidine (4.3 g) as a colourless solid.

b) Methyl 2-/4-(5-chloro-N-acetyl-2-pyrimidylamino) phenoxy/propionate was prepared from 5-chloro-2-(N-acetyl-4-methoxyphenylamino)pyrimidine and methyl 2-bromopropionate following essentially the same procedure as that described in Example 3 parts b) and c). The product, a colourless oil, was characterised by its p.m.r. spectrum (chemical shift δ in ppm): 1.6, d(3H); 2.2, s(3H); 3.7, s(3H); 4.8, q(1H); 7.1, d of d(4H); 8.6, s(2H).

Example 7

Preparation of Methyl 2-/4-(5-trifluoromethyl-N-methyl-2-pyrimidylamino)phenoxy/propionate (7)

a) A mixture of 4-N-methylaminophenol sulfate (2.8 g), 2-chloro-5-trifluoromethylpyrimidine (3.0 g) and water (50 ml) was heated under reflux for a period of 2 hr. The reaction mixture was cooled and extracted with dichloromethane (2 x 200 ml) to give 4-(N-methyl-5-trifluoromethyl-2-pyrimidylamino)phenol as an oil (2.0 g) which solidified on standing.

b) Methyl 2-/4-(5-trifluoromethyl-N-methyl-2-pyrimidylamino)phenoxy/propionate was prepared from 4-(N-methyl-5-trifluoromethyl)-2-pyrimidylamino)phenol and methyl 2-bromopropionate following essentially the same procedure as that described in Example 1 part c). The product was a colourless solid, mp 80°C.

Example 8

The following compounds were prepared from the appropriate 2-chloropyrimidine, the appropriate 4-N-alkylaminophenol sulfate and methyl 2-bromopropionate following essentially the same procedure as that described in Example 7.

a) Methyl 2-[4-(4,6-dimethyl-N-methyl-2-pyrimidylamino)-phenoxy]propionate (8) was obtained as an oil and was characterised by its p.m.r. spectrum (chemical shift δ in ppm): 1.6, d(3H); 2.3, s(6H); 3.6, s(3H); 3.9, s(3H); 4.8 q(1H); 6.4, s(1H); 7.2, d of d(4H).

b) Methyl 2-[4-(N-ethyl-5-trifluoromethyl-2-pyrimidyl-amino)phenoxy]propionate (9) was obtained as a colourless solid, mp 45°C.

c) Methyl 2-[4-(5-methyl-N-methyl-2-pyrimidylamino)-phenoxy]propionate (10) was obtained as a colourless solid, mp 71°C.

d) Methyl 2-[4-(5-bromo-N-methyl-2-pyrimidylamino)-phenoxy]propionate (11) was obtained as a colourless solid, mp 81°C.

e) Methyl 2-[4-(5-bromo-N-ethyl-2-pyrimidylamino)-phenoxy]propionate (12) was obtained as a colourless oil and was characterised by its p.m.r. spectrum (chemical shift δ in ppm): 1.1, t(3H); 1.5, d(3H); 3.7, s(3H); 3.9 q(1H); 4.7, q(1H); 6.9, d of d(4H); 8.2, s(2H).

f) Methyl 2-[4-(N-methyl-2-pyrimidylamino)phenoxy]-propionate (14) was obtained as a colourless solid, mp 91°C.

g) Methyl 2-[4-(N-methyl-5-phenyl-2-pyrimidylamino)-phenoxy]propionate (17) was obtained as a colourless oil and was characterised by its p.m.r. spectrum

(chemical shift δ in ppm): 1.6, d(3H); 3.5, s(3H); 3.8, s(3H); 4.8, q(1H); 6.8-7.6, m(9H); 8.6, s(2H).

Example 9

Preparation of Methyl 2-/3-chloro-4-(5-bromo-2-pyrimidylamino)phenoxy/propionate (13)

A mixture of methyl 2-/4-(5-bromo-2-pyrimidylamino)phenoxy/propionate (1.0 g, Example 3), N-chlorosuccinimide (0.4 g) and chloroform (5 ml) was stirred at a temperature of 20°C for a period of 16 hr. Water was added and the aqeuous mixture was extracted with chloroform (2 x 50 ml). The combined extracts were dried over anhydrous magnesium sulfate and the chloroform was evaporated from the dried solution to give methyl 2-/3-chloro-4-(5-bromo-2-pyrimidylamino)phenoxy/-propionate (0.82 g) as a colourless solid, mp 103°C.

Example 10

Methyl 2-/4-(N-methyl-5-nitro-2-pyrimidylamino)phenoxy/-propionate (15) was prepared from 1,3-bis(dimethylamino)-2-nitrotrimethinium perchlorate /ref. Coll. Czech. Chem. Comm., 32, 1704 (1967)/, 1-methyl-1-(4-hydroxyphenyl)-guanidine sulfate and methyl 2-bromopropionate following essentially the same procedure as that described in Example 1 parts b) and c). The product was obtained as a pale yellow solid, mp 127°C, after crystallisation from ethanol.

Example 11

Preparation of 2-/4-(5-Bromo-2-pyrimidylamino)phenoxy/-propionic acid (16)

A solution of methyl 2-/4-(5-bromo-2-pyrimidylamino)phenoxy/propionate (1.1 g; see Example 3) and potassium hydroxide (0.2 g) in methanol (20 ml) was allowed to stand at a temperature of 20°C for a period of 24 hours. The methanol was removed by evaporation under reduced presure and the residue was partitioned

between chloroform (100 ml) and dilute aqueous hydrochloric acid. The chloroform was removed by evaporation under reduced pressure to give 2-/4-(5-bromo-2-pyrimidylamino)phenoxy/propionic acid (0.4 g), mp 215°C (decomp.).

Example 12

Preparation of n-Butyl 2-/4-(5-bromo-N-methyl-2-pyrimidylamino)phenoxy/propionate (18)

a) 2-/4-(5-Bromo-N-methyl-2-pyrimidylamino)phenoxy/-propionic acid was prepared by the hydrolysis of the methyl ester (see Example 8 part d)) following essentially the same procedure as that described in Example 11.

b) A mixture of 2-/4-(5-bromo-N-methyl-2-pyrimidylamino)phenoxy/propionic acid (1.8 g) and thionyl chloride (10 ml) was heated under reflux for a period of 3 hours. The excess thionyl chloride was removed by evaporation under reduced pressure and n-butanol (1 ml) was added to the residue. After standing at a temperature of 20°C for a period of 18 hours the mixture was chromatographed over silica gel with chloroform elution to give n-butyl 2-/4-(5-bromo-N-methyl-2-pyrimidylamino)phenoxy/propionate (0.7 g) as a colourless oil.
Proton magnetic resonance spectrum ($CDCl_3$; $\delta$ in ppm): 0.8-1.7, m(10H); 3.5, s(3H); 4.2, t(2H); 4.8, q(1H); 7.1, d of d(4H); 8.4, s(2H).

Example 13

Preparation of 2-{4-/5-Bromo-N-(1-propyl)-2-pyrimidylamino/phenoxy}propionic acid (19)

A mixture of methyl 2-/4-(5-bromo-2-pyrimidylamino)phenoxy/propionate (1.3 g; see Example 3), potassium tertiarybutoxide (0.5 g) and dimethylformamide (20 ml), stirred at a temperature of 20°C, was treated with n-propyl iodide (0.7 g). After stirring for

a period of 18 hours the mixture was poured into water (100 ml) and the aqueous solution was extracted with dichloromethane. The dichloromethane extracts were dried (anhydrous $MgSO_4$) and the solvent was removed by evaporation under reduced pressure to give a dark oil. The oil was chromatographed over silica gel (30 g) with chloroform elution to give 2-{4-/5-bromo-N-(1-propyl)-2-pyrimidylamino/phenoxy}propionic acid (0.22 g) as pale brown crystals.

Proton magnetic resonance spectrum (chemical shift δ in ppm): 0.9, t(3H); 1.6, m(5H); 3.8 t(2H); 4.8, q(1H); 7.0, m(4H); 8.3, s(2H).

Example 14

2-(N,N-Dimethylamino)ethyl 2-/4-(5-bromo-N-methyl-2-pyrimidylamino)phenoxy/propionate (20) was prepared from 2-/4-(5-bromo-N-methyl-2-pyrimidylamino)phenoxy/propionic acid and 2-(N,N-dimethylamino)ethanol following essentially the same procedure as that described in Example 12 for the preparation of n-butyl 2-/4-(5-bromo-N-methyl-2-pyrimidylamino)phenoxy/propionate.

Proton magnetic resonance spectrum (chemical shift δ in ppm): 1.6, d(3H); 2.2, s(6H); 2.5, t(2H); 3.5, s(3H); 4.3, t(2H); 4.8, q(1H); 7.1, d of d(4H); 8.4, s(2H).

Example 15

Preparation of 2-(N,N,N-trimethylammonio)ethyl 2-/4-(5-bromo-N-methyl-2-pyrimidylamino)phenoxy/propionate iodide salt (21)

A mixture of 2-(N,N-dimethylamino)ethyl 2-/4-(5-bromo-N-methyl-2-pyrimidylamino)phenoxy/propionate (1.24 g; see Example 14) dichloromethane (50 ml) and methyl iodide (1 ml) was stirred for 3 days at a temperature of 20°C. The white precipitate which formed was collected by filtration to give 2-(N,N,N-trimethylammonio)ethyl 2-/4-(5-bromo-N-methyl-2-pyrimidylamino)-

phenoxy]propionate (1.3 g), mp 125-130°C.

Example 16

Concentrated formulations of the compounds of the invention were prepared by:

a) in the case of oils and waxy solids, dissolving the compound in toluene containing 7% v/v "Teric" N13 ("Teric" is a Trade Mark and "Teric" N13, a product of ethoxylation of nonylphenol, is available from ICI Australia Limited) and 3% v/v "Kemmat" SC15B ("Kemmat" is a Trade Mark and "Kemmat" SC15B is a formulation of calcium dodecylbenzene sulfonate); or

b) in the case of crystalline solids, adding 5 parts by weight of the compound and 1 part by weight of "Dyapol" PT ("Dyapol" is a Trade Mark and "Dyapol" PT is an anionic suspending agent) to 94 parts by weight of an aqueous solution containing 0.25% v/v of "Teric" N8 (a product of ethoxylation of nonylphenol) and ball-milling the mixture to produce a stable suspension.

The emulsifiable concentrates and suspensions were then diluted with water to give an aqueous composition of the required concentration suitable for use in the evaluation of the pre-emergence and post-emergence herbicidal activity of the compounds of the invention.

Example 17

The pre-emergent herbicidal activity of the compounds of the invention formulated as described in Example 16 was assessed by the following procedure.

The seeds of the test species were sown in rows 2 cm deep in soil contained in seed boxes. The monocotyledonous plants and the dicotyledonous plants were sown in separate boxes and after sowing the two boxes were sprayed with the required quantity of a composition of the invention. Two duplicate seed boxes were prepared in the same manner but were not sprayed with a composition of the invention and were used for comparison purposes. All the boxes were placed in a glasshouse, lightly watered with an overhead spray to initiate germination and then sub-irrigated as required for optimum plant growth. After three weeks the boxes were removed from the glasshouse and the effect of the treatment was visually assessed. The results are presented in Table 2 where the damage to plants is rated on a scale of from 0 to 3 where 0 represents from 0 to 25% damage, 3 represents 75 to 99% kill and 3+ represents 100% kill.

A dash (-) means that no experiment was carried out.

The names of the test plants are as follows:

| | |
|---|---|
| Wh | Wheat |
| Ot | Wild Oats |
| Rg | Ryegrass |
| Jm | Japanese millet |
| P | Peas |
| Ip | Ipomea |
| Ms | Mustard |
| Sf | Sunflower |

TABLE 2

PRE-EMERGENCE HERBICIDAL ACTIVITY

| Compound No | APPLICATION Rate (kg/ha) | TEST PLANT Wh | Ot | Rg | Jm | P | Ip | Ms | Sf |
|---|---|---|---|---|---|---|---|---|---|
| 1 | 5 | 0 | 0 | 3 | 3 | 0 | 0 | 0 | 0 |
| 2 | 5 | 3 | 3 | 3 | 3+ | 0 | 0 | 0 | 0 |
| 2 | 1 | 2 | 3+ | 3+ | 3+ | 0 | 0 | 0 | 0 |
| 2 | 0.5 | 2 | 2 | 2 | 3 | 0 | 0 | 0 | 0 |
| 3 | 1.0 | 2 | 2 | 3 | 3+ | 0 | 0 | 0 | 0 |
| 3 | 0.5 | 1 | 0 | 3 | 3 | 0 | 0 | 0 | 0 |
| 7 | 2 | 3+ | 3+ | 3+ | 3+ | 3 | 0 | 0 | 0 |
| 7 | 1 | 3+ | 3 | 3 | 3+ | 0 | 0 | 0 | 0 |
| 7 | 0.5 | 3 | 3 | 3 | 3+ | 1 | 0 | 0 | 0 |
| 9 | 5 | 3 | 3+ | 3+ | 3+ | 0 | 0 | 0 | 0 |
| 9 | 1 | 2 | 2 | 3 | 3 | 0 | 0 | 0 | 0 |
| 9 | 0.5 | 2 | 2 | 2 | 3 | 0 | 0 | 0 | 0 |
| 11 | 5 | 3 | 3+ | 3+ | 3+ | 0 | 0 | 0 | 0 |
| 11 | 1 | 1 | 2 | 3 | 3 | 0 | 0 | 0 | 0 |
| 12 | 5 | 2 | 2 | 3+ | 3+ | 0 | 0 | 0 | 0 |
| 16 | 5 | 1 | 1 | 3+ | 3+ | 0 | 0 | 0 | 0 |
| 17 | 5 | 0 | 0 | 0 | 2 | 0 | 0 | 0 | 0 |
| 17 | 1 | 0 | 0 | 0 | 0 | 0 | 0 | 0 | 0 |
| 18 | 5 | 2 | 2 | 3 | 3+ | 0 | 0 | 0 | 0 |
| 18 | 1 | 0 | 0 | 2 | 2 | 0 | 0 | 0 | 0 |
| 20 | 5 | 3 | 3 | 3 | 3+ | 0 | 0 | 0 | 0 |
| 20 | 1 | 2 | 1 | 2 | 3 | 0 | 0 | 0 | 0 |
| 21 | 4 | 3 | 3 | 3 | 3+ | 0 | 0 | 0 | 0 |

Example 18

The post-emergent herbicidal activity of the compounds of the invention formulated as described in Example 16 was assessed by the following procedure.

The seeds of the test species were sown in rows 2 cm deep in soil contained in seed boxes. The monocotyledonous plants and the dicotyledonous plants were sown in separate seed boxes in duplicate. The four seed boxes were placed in a glasshouse, lightly watered with an overhead spray to initiate germination and then sub-irrigated as required for optimum plant growth. After the plants had grown to a height of about 10 to 12.5 cm one box of each of the monocotyledonous plants and the dicotyledonous plants was removed from the glasshouse and sprayed with the required quantity of a composition of the invention. After spraying the boxes were returned to the glasshouse for further 3 weeks and the effects of treatment was visually assessed by comparison with the untreated controls. The results are presented in Table 3 where the damage to plants is rated on a scale of from 0 to 3 where 0 represents 0 to 25% damage, 3 represents 75 to 99% kill and 3+ represents 100% kill. A dash (-) means that no experiment was carried out.

The names of the test plants are as follows:

| | |
|---|---|
| Wh | Wheat |
| Ot | Wild Oats |
| Rg | Ryegrass |
| Jm | Japanese millet |
| P | Peas |
| Ip | Ipomea |
| Ms | Mustard |
| Sf | Sunflower |

TABLE 3

POST-EMERGENCE HERBICIDAL ACTIVITY

| Compound No | APPLICATION Rate (kg/ha) | TEST PLANT | | | | | | | |
|---|---|---|---|---|---|---|---|---|---|
| | | Wh | Ot | Rg | Jm | P | Ip | Ms | Sf |
| 1 | 5 | 0 | 0 | 1 | 3 | 0 | 2 | 3 | 3 |
| 2 | 5 | 3 | 3+ | 3+ | 3+ | 1 | 1 | 1 | 1 |
| 2 | 1 | 3 | 3+ | 3 | 3+ | 0 | 0 | 0 | 0 |
| 2 | 0.5 | 2 | 2 | 3 | 3+ | 0 | 0 | 0 | 0 |
| 3 | 1 | 3+ | 1 | 3 | 3+ | 0 | 0 | 0 | 0 |
| 3 | 0.5 | 3 | 1 | 3 | 3+ | 0 | 0 | 0 | 0 |
| 7 | 2 | 3+ | 3+ | 3+ | 3+ | 3+ | 2 | 3 | 2 |
| 7 | 1 | 3+ | 3+ | 3+ | 3+ | 2 | 1 | 2 | 2 |
| 7 | 0.5 | 3 | 3+ | 3+ | 3+ | 0 | 0 | 1 | 2 |
| 9 | 5 | 3 | 3 | 3+ | 3+ | 3 | 0 | 0 | 3 |
| 9 | 1 | 3 | 3 | 3 | 3+ | 0 | 0 | 0 | 0 |
| 9 | 0.5 | 2 | 2 | 2 | 2 | 0 | 0 | 0 | 0 |
| 11 | 5 | 3+ | 3+ | 3+ | 3+ | 0 | 1 | 2 | 3 |
| 11 | 1 | 3 | 3+ | 3+ | 3+ | 0 | 0 | 0 | 0 |
| 11 | 0.5 | 2 | 3 | 3 | 3+ | 0 | 0 | 0 | 0 |
| 12 | 5 | 3+ | 3 | 3+ | 3+ | 2 | 1 | 3 | - |
| 12 | 1 | 3 | 2 | 3 | 3+ | 0 | 0 | 0 | 2 |
| 16 | 5 | 0 | 1 | 0 | 2 | 3+ | 0 | 0 | 0 |
| 17 | 5 | 3 | 3 | 3+ | 3+ | 0 | 0 | 0 | 0 |
| 17 | 1 | 3 | 2 | 2 | 3+ | 0 | 0 | 0 | 0 |
| 18 | 5 | 3+ | 3+ | 3+ | 3+ | 0 | 2 | 2 | 2 |
| 18 | 1 | 3 | 3 | 3 | 3+ | 0 | 0 | 0 | 0 |
| 20 | 5 | 3+ | 3+ | 3+ | 3+ | 2 | 1 | 3 | 2 |
| 20 | 1 | 2 | 3+ | 3 | 3+ | 0 | 0 | 0 | 0 |
| 21 | 4 | 3+ | 3+ | 3+ | 3+ | 2 | 3 | 3+ | 2 |

Example 19

The compounds were formulated for test by mixing an appropriate amount with 5 ml of an emulsion prepared by diluting 160 ml of a solution containing 21.8 g per litre of "Span" 80 and 78.2 g per litre of "Tween" 20 in methylcyclohexanone to 500 ml with water. "Span" 80 is a Trade Mark for a surface-active agent comprising sorbitan monolaurate. "Tween" 20 is a Trade Mark for a surface-active agent comprising a condensate of sorbitan monolaurate with 20 molar proportions of ethylene oxide. Each 5 ml emulsion containing a test compound was then diluted to 40 ml with water and sprayed on to young pot plants (post-emergence test) of the species named in Table 4 below. Damage to test plants was assessed after 14 days on a scale of 0 to 5 where 0 is 0 to 20% damage and 5 is complete kill. In a test for pre-emergence herbicidal activity, seeds of the test plants were sown in a shallow slit formed in the surface of soil in fibre trays. The surface was then levelled and sprayed, and fresh soil then spread thinly over the sprayed surface. Assessment of herbicidal damage was carried out after 21 days using the same scale of 0 to 5 as the post-emergence test. In both cases the degree of herbicidal damage was assessed by comparison with untreated control plants. The results are given in Table 4 below. A dash (-) means that no experiment was carried out.

The names of the test plants were as follows:

| | |
|---|---|
| Sb | Sugar beet |
| Rp | Rape |
| Ct | Cotton |
| Sy | Soy bean |
| Mz | Maize |
| Mw | Winter wheat |
| Rc | Rice |
| Sn | *Senecio vulgaris* |
| Ip | *Ipomea purpurea* |
| Am | *Amaranthus retroflexus* |
| Pi | *Polygonum aviculare* |
| Ca | *Chenopodium album* |
| Po | *Portulaca oleracea* |
| Xa | *Xanthium pensylvanicum* |
| Ab | *Abutilon theophrasti* |
| Cv | *Convolvulus arvensis* |
| Ot | Cultivated oats and wild oats (*Avena fatua*) Wild oats are used in the post-emergence test and cultivated oats in the pre-emergence test |
| Dg | *Digitaria sanguinalis* |
| Pu | *Poa annua* |
| St | *Setaria viridis* |
| Ec | *Echinochloa crus-galli* |
| Sh | *Sorghum halepense* |
| Ag | *Agropyron repens* |
| Cn | *Cyperus rotundus* |

TABLE 4 - PART A

| Compound No | APPLICATION Method | Rate (kg/ha) | TEST PLANT Sb | Rp | Ct | Sy | Mz | Ww | Rc | Sn | Ip | Am | Pi | Ca |
|---|---|---|---|---|---|---|---|---|---|---|---|---|---|---|
| 2 | PRE | 2.0 | 3 | 0 | 0 | 1 | 5 | 5 | 4 | 2 | 1 | - | - | - |
| 2 | PRE | 0.5 | 0 | 0 | 0 | 0 | 3 | 3 | 3 | 2 | 0 | - | - | - |
| 2 | PRE | 0.25 | 3 | 0 | 0 | 0 | 3 | 0 | 0 | 3 | 0 | 1 | - | 0 |
| 2 | POST | 2.0 | 3 | 2 | 2 | 2 | 5 | 4 | 2 | 4 | 1 | 2 | 4 | 3 |
| 2 | POST | 0.5 | 2 | 1 | 0 | 2 | 4 | 2 | 2 | 3 | 0 | 2 | 3 | 3 |
| 2 | POST | 0.25 | 2 | 2 | 0 | 2 | 4 | 4 | 3 | 2 | 0 | 2 | 2 | 3 |
| 3 | PRE | 1.0 | 1 | 0 | 0 | 1 | 4 | 5 | 3 | 3 | - | 1 | - | 0 |
| 3 | PRE | 0.25 | 1 | 0 | - | 0 | 2 | 2 | 2 | 3 | 0 | 1 | - | 0 |
| 3 | POST | 1.0 | 2 | 3 | 4 | 4 | 4 | 4 | 4 | 5 | 0 | 4 | 4 | 3 |
| 3 | POST | 0.25 | 0 | 1 | 0 | 2 | 3 | 4 | 2 | 3 | 0 | 1 | 1 | 1 |
| 5 | PRE | 2.0 | 1 | 0 | 0 | 2 | 0 | 0 | 0 | 0 | 0 | - | - | - |
| 5 | PRE | 0.5 | 1 | 0 | 0 | 0 | - | 0 | 0 | 0 | 0 | - | - | - |
| 5 | POST | 2.0 | 4 | 2 | 3 | 4 | 0 | 1 | 0 | 4 | 2 | 4 | 4 | 4 |
| 5 | POST | 0.5 | 3 | 2 | 0 | 2 | 0 | 0 | 0 | 4 | 0 | 4 | 2 | 4 |
| 6 | PRE | 2.0 | 1 | 3 | 2 | 3 | 0 | 0 | 0 | 2 | 0 | - | - | - |
| 6 | PRE | 0.5 | 1 | 1 | 0 | 1 | 0 | 0 | 0 | 2 | 0 | - | - | - |
| 6 | POST | 2.0 | 4 | 3 | 0 | 4 | 4 | 1 | 1 | 4 | 2 | 1 | 3 | 3 |
| 6 | POST | 0.5 | 2 | 2 | 0 | 0 | 0 | 0 | 0 | 4 | 0 | 0 | 2 | 2 |
| 7 | PRE | 1.0 | 0 | 0 | 0 | 1 | 5 | 4 | 4 | 0 | 2 | 1 | - | 4 |
| 7 | PRE | 0.25 | 2 | 0 | 0 | 0 | 4 | 5 | 0 | 0 | 0 | - | - | 0 |
| 7 | POST | 1.0 | 3 | 3 | 2 | 4 | 5 | 5 | 4 | 4 | 0 | 1 | 3 | 3 |
| 7 | POST | 0.25 | 1 | 3 | 0 | 3 | 5 | 4 | 2 | 3 | 0 | 1 | 2 | 2 |
| 9 | PRE | 2.0 | 1 | 1 | 1 | 0 | 5 | 4 | 3 | 0 | 0 | - | - | - |
| 9 | PRE | 0.5 | - | 0 | 0 | 0 | 2 | 0 | 0 | 0 | 0 | - | - | - |
| 9 | POST | 2.0 | 1 | 2 | 1 | 4 | 4 | 3 | 2 | 4 | 0 | 3 | 4 | 4 |
| 9 | POST | 0.5 | 1 | 2 | 2 | 1 | 4 | 4 | 1 | 2 | 0 | 0 | 3 | 0 |

TABLE 4 - PART A Continued

| Compound No | APPLICATION Method | Rate (kg/ha) | TEST PLANT Sb | Rp | Ct | Sy | Mz | Ww | Rc | Sn | Ip | Am | Pi | Ca |
|---|---|---|---|---|---|---|---|---|---|---|---|---|---|---|
| 11 | PRE | 2.0 | 0 | 0 | 1 | 1 | 5 | 5 | 5 | 0 | 0 | - | - | - |
| 11 | PRE | 0.5 | 0 | 0 | 0 | 0 | 3 | 4 | 5 | 0 | 0 | - | - | - |
| 11 | POST | 2.0 | 4 | 3 | 3 | 4 | 5 | 4 | 3 | 4 | 0 | 4 | 4 | 4 |
| 11 | POST | 0.5 | 2 | 2 | 1 | 2 | 5 | 3 | 2 | 4 | 0 | 1 | 4 | 3 |
| 13 | PRE | 0.5 | 0 | 1 | - | 0 | 0 | 0 | 0 | 0 | 0 | 0 | - | - |
| 13 | POST | 0.5 | 1 | 2 | 0 | 1 | 1 | 2 | 0 | 2 | 0 | 2 | - | 0 |
| 15 | PRE | 5.0 | 0 | 0 | 0 | 0 | 2 | 2 | 3 | 0 | 0 | 1 | 0 | 1 |
| 15 | PRE | 1.0 | 0 | 0 | 0 | 0 | 1 | 0 | 0 | 0 | 0 | 0 | 0 | 0 |
| 18 | PRE | 2.0 | 0 | 0 | 0 | 0 | 5 | 5 | 5 | 0 | 0 | 1 | 0 | 2 |
| 18 | PRE | 0.5 | 0 | 0 | 0 | 0 | 3 | 4 | 2 | 0 | 0 | 2 | 1 | 1 |
| 18 | POST | 2.0 | 3 | 3 | 1 | 4 | 5 | 4 | 4 | 4 | 0 | 4 | 3 | - |
| 18 | POST | 0.5 | 2 | 2 | 0 | 0 | 5 | 4 | 2 | 3 | 0 | 3 | 2 | - |

TABLE 4 - PART B

| Compound No | APPLICATION Method | Rate (kg/ha) | Po | Xa | Ab | Cv | Ot | Dg | Pu | St | Ec | Sh | Ag | Cn |
|---|---|---|---|---|---|---|---|---|---|---|---|---|---|---|
| 2 | PRE | 2.0 | - | 2 | 1 | 1 | 4 | 4 | 0 | 5 | 5 | 4 | 5 | 2 |
| 2 | PRE | 0.5 | - | 0 | 1 | 0 | 2 | 4 | 0 | 4 | 4 | 4 | 2 | 0 |
| 2 | PRE | 0.25 | - | 0 | 0 | - | 0 | 4 | 2 | 3 | 2 | 2 | 1 | 0 |
| 2 | POST | 2.0 | 1 | 2 | 2 | 0 | 5 | 5 | 0 | 5 | 5 | 5 | 3 | 0 |
| 2 | POST | 0.5 | 0 | 1 | 2 | 0 | 3 | 1 | 0 | 4 | 5 | 3 | 2 | 0 |
| 2 | POST | 0.25 | 5 | 0 | 0 | 1 | 4 | 3 | 0 | 4 | 5 | 4 | 2 | 0 |
| 3 | PRE | 1.0 | 4 | 0 | 2 | - | 2 | 4 | 2 | 4 | 3 | 3 | 4 | 0 |
| 3 | PRE | 0.25 | 0 | 1 | 2 | - | 0 | 4 | 2 | 3 | 1 | 2 | 1 | 0 |
| 3 | POST | 1.0 | 1 | 1 | 2 | 0 | 4 | 5 | 2 | 4 | 5 | 4 | 4 | 0 |
| 3 | POST | 0.25 | 0 | 0 | 0 | 0 | 4 | 2 | 0 | 5 | 5 | 3 | 3 | 0 |
| 5 | PRE | 2.0 | 1 | 0 | 0 | - | 0 | 1 | 0 | 0 | 0 | - | 0 | 0 |
| 5 | PRE | 0.5 | 2 | 0 | 0 | - | 0 | 0 | 0 | 0 | 1 | - | 0 | 0 |
| 5 | POST | 2.0 | 4 | 1 | 3 | 1 | 1 | 3 | 1 | 2 | 0 | 0 | 0 | 0 |
| 5 | POST | 0.5 | 3 | 1 | 2 | 0 | 0 | 4 | 0 | 0 | 0 | 0 | 0 | 0 |
| 6 | PRE | 2.0 | 5 | 1 | 0 | - | 0 | 2 | 0 | 0 | 0 | 2 | 0 | 0 |
| 6 | PRE | 0.5 | 2 | 1 | 0 | - | 0 | 1 | 0 | 0 | 0 | 1 | 0 | 0 |
| 6 | POST | 2.0 | 0 | 2 | 2 | 0 | 1 | 5 | 3 | 0 | 2 | 0 | 0 | 0 |
| 6 | POST | 0.5 | 0 | 1 | 1 | 0 | 0 | 1 | 0 | 0 | 1 | 0 | 0 | 0 |
| 7 | PRE | 1.0 | 0 | 0 | 0 | - | 4 | 4 | - | 4 | 5 | - | 5 | 0 |
| 7 | PRE | 0.25 | 0 | 0 | 0 | - | 4 | 0 | - | 3 | 4 | - | 1 | 0 |
| 7 | POST | 1.0 | 1 | 0 | 3 | 0 | 5 | 5 | 4 | 5 | 5 | 5 | 5 | 0 |
| 7 | POST | 0.25 | 1 | 0 | 2 | 0 | 4 | 2 | 2 | 4 | 5 | 5 | 5 | 0 |
| 9 | PRE | 2.0 | 1 | 1 | 0 | - | 3 | 5 | 0 | 4 | 4 | 4 | 4 | 1 |
| 9 | PRE | 0.5 | 1 | 1 | 0 | - | 0 | 4 | 0 | 2 | 1 | 2 | 0 | 0 |
| 9 | POST | 2.0 | 2 | 0 | 1 | 0 | 4 | 4 | 0 | 4 | 4 | 5 | 2 | 0 |
| 9 | POST | 0.5 | 0 | 0 | 0 | 0 | 4 | 3 | 0 | 4 | 4 | 4 | 2 | 0 |

TABLE 4 - PART B Continued

| Compound No | APPLICATION Method | Rate (kg/ha) | TEST PLANT Po | Xa | Ab | Cv | Ot | Dg | Pu | St | Ec | Sh | Ag | Cn |
|---|---|---|---|---|---|---|---|---|---|---|---|---|---|---|
| 11 | PRE | 2.0 | 0 | 0 | 0 | - | 4 | 4 | 1 | 4 | 5 | 4 | 5 | 0 |
| 11 | PRE | 0.5 | - | 0 | 0 | - | 3 | 4 | 0 | 4 | 3 | 4 | 4 | 0 |
| 11 | POST | 2.0 | 2 | 0 | 4 | 0 | 5 | 5 | 1 | 5 | 5 | 5 | 4 | 0 |
| 11 | POST | 0.5 | 0 | 0 | 1 | 0 | 4 | 3 | 0 | 5 | 5 | 5 | 4 | 0 |
| 13 | PRE | 0.5 | 1 | 1 | 0 | - | 0 | - | 0 | 0 | 0 | 1 | 0 | 0 |
| 13 | POST | 0.5 | 0 | 0 | 0 | 0 | 0 | 2 | 0 | 2 | 0 | 0 | 0 | 0 |
| 15 | PRE | 5.0 | 1 | 0 | 0 | - | 1 | 4 | 1 | 4 | 4 | 4 | 0 | 0 |
| 15 | PRE | 1.0 | 0 | 0 | 0 | - | 0 | 0 | 0 | 0 | 0 | 2 | 0 | 0 |
| 18 | PRE | 2.0 | 0 | 0 | 0 | - | 4 | 4 | 0 | 5 | 5 | 4 | 5 | 0 |
| 18 | PRE | 0.5 | 0 | - | 0 | - | 3 | 4 | 0 | 4 | 4 | 4 | 1 | 0 |
| 18 | POST | 2.0 | 0 | 1 | 4 | 0 | 4 | 5 | 0 | 5 | 5 | 4 | 4 | 0 |
| 18 | POST | 0.5 | 0 | 0 | 1 | 0 | 4 | 0 | 0 | 4 | 5 | 2 | 3 | 0 |

1. A compound of formula I

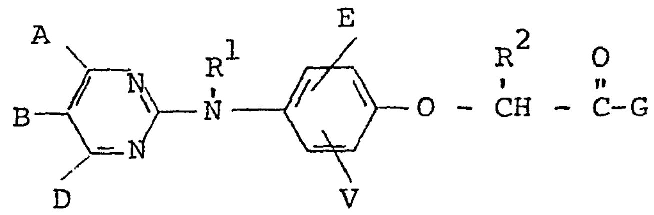

I

or a salt thereof wherein:

A, B, D, E and V are independently chosen from the group consisting of hydrogen, halogen, nitro, cyano, thiocyano, amino, $C_1$ to $C_6$ alkylamino, di($C_1$ to $C_6$ alkyl)amino, $C_1$ to $C_6$ alkyl, $C_1$ to $C_6$ haloalkyl, $C_2$ to $C_6$ alkenyl, $C_3$ to $C_7$ cycloalkyl, $C_1$ to $C_6$ alkoxy, $C_1$ to $C_6$ haloalkoxy, $C_1$ to to $C_6$ alkylthio, $C_1$ to $C_6$ alkylsulfinyl, $C_1$ to $C_6$ alkylsulfonyl, $C_1$ to $C_6$ haloalkylsulfinyl, $C_1$ to $C_6$ haloalkylsulfonyl, sulfo, $C_1$ to $C_6$ alkoxysulfonyl, sulfamoyl, N-($C_1$ to $C_6$ alkyl) sulfamoyl, N,N-di($C_1$ to $C_6$ alkyl)-sulfamoyl, carboxy, ($C_1$ to $C_6$ alkoxy)carbonyl, carbamoyl, N-($C_1$ to $C_6$ alkyl)carbamoyl, N,N-di($C_1$ to $C_6$ alkyl)-carbamoyl, phenyl, phenoxy, phenylthio, and the groups substituted phenyl, substituted phenoxy and substituted phenylthio wherein in each group the phenyl ring is substituted with one to three substituents chosen from the group consisting of halogen, $C_1$ to $C_6$ alkyl, $C_1$ to $C_6$ haloalkyl, $C_1$ to $C_6$ alkoxy, nitro and cyano;

$R^1$ is chosen from the group consisting of hydrogen, $C_1$ to $C_{10}$ alkyl, $C_2$ to $C_{10}$ alkenyl, $C_2$ to $C_{10}$ alkoxyalkyl, $C_1$ to $C_{10}$ haloalkyl, formyl, $C_2$ to $C_{10}$ alkanoyl, phenyl, benzyl, benzoyl, and the groups phenyl, benzyl and benzoyl wherein in each group the phenyl ring is substituted with one or two substituents chosen from the group consisting of halogen, $C_1$ to $C_6$ alkyl, $C_1$ to $C_6$ haloalkyl, $C_1$ to $C_6$ alkoxy, nitro and cyano;

$R^2$ is chosen from the group consisting of hydrogen, $C_1$ to $C_6$ alkyl, $C_2$ to $C_6$ alkenyl, $C_2$ to $C_6$ alkoxyalkyl,

$C_1$ to $C_6$ haloalkyl, acetyl, propionyl, and $C_2$ to $C_6$ alkoxycarbonyl; and

G is chosen from the group consisting of hydroxy, mercapto, $C_1$ to $C_{10}$ alkoxy, $C_1$ to $C_{10}$ haloalkoxy, $C_2$ to $C_{10}$ alkenyloxy, $C_2$ to $C_{10}$ alkynyloxy, $C_1$ to $C_{10}$ alkylthio, $C_2$ to $C_{10}$ alkenylthio, $C_2$ to $C_{10}$ alkynylthio, $C_3$ to $C_7$ cycloalkoxy, $C_3$ to $C_7$ cycloalkoxy substituted with one or two $C_1$ to $C_4$ alkyl groups, phenoxy, phenylthio, benzyloxy, benzylthio, the group $C_1$ to $C_{10}$ alkoxy substituted with a substituent chosen from the group consisting of $C_1$ to $C_6$ alkoxy, amino, ammonio, cyano, N-($C_1$ to $C_6$ alkyl)amino, N,N-di($C_1$ to $C_6$ alkyl)amino, and N,N,N-tri($C_1$ to $C_6$ alkyl)-ammonio, the groups phenoxy, phenylthio, benzyloxy and benzylthio wherein in each group the phenyl ring is substituted with one or two substituents chosen from the group consisting of halogen, nitro, cyano, $C_1$ to $C_6$ alkyl, $C_1$ to $C_6$ haloalkyl and $C_1$ to $C_6$ alkoxy, the group OM wherein M is the cation of an inorganic or organic base, the group $-NHSO_2R^3$ wherein $R^3$ is chosen from $C_1$ to $C_{10}$ alkyl and $C_1$ to $C_{10}$ haloalkyl, and the group $-NR^4R^5$ wherein $R^4$ and $R^5$ are independently chosen from the group consisting of hydrogen, $C_1$ to $C_6$ alkyl, phenyl and benzyl or $R^4$ and $R^5$ together form a heterocyclic ring.

2. A compound according to claim 1 wherein:

A, B, D, E and V are independently chosen from the group consisting of hydrogen, halogen, nitro, cyano, thiocyano, amino, $C_1$ to $C_6$ alkylamino, di($C_1$ to $C_6$ alkyl)amino, $C_1$ to $C_6$ alkyl, $C_1$ to $C_6$ haloalkyl, $C_2$ to $C_6$ alkenyl, $C_3$ to $C_7$ cycloalkyl, $C_1$ to $C_6$ alkoxy, $C_1$ to $C_6$ alkylthio, ($C_1$ to $C_6$ alkoxy)carbonyl, phenyl, phenoxy, phenylthio, and the groups substituted phenyl, substituted phenoxy and substituted phenylthio wherein in each group the phenyl ring is substituted with one to three substituents chosen from the group consisting of halogen, $C_1$ to $C_6$ alkyl, $C_1$ to $C_6$ haloalkyl, $C_1$ to $C_6$ alkoxy, nitro and

cyano;
$R^1$ is chosen from the group consisting of hydrogen, $C_1$ to $C_{10}$ alkyl, $C_2$ to $C_{10}$ alkenyl, $C_2$ to $C_{10}$ alkoxyalkyl, $C_1$ to $C_{10}$ haloalkyl, formyl, $C_2$ to $C_{10}$ alkanoyl, phenyl, benzyl, benzoyl, and the groups phenyl, benzyl and benzoyl wherein in each group the phenyl ring is substituted with one or two substituents chosen from the group consisting of halogen, $C_1$ to $C_6$ alkyl, $C_1$ to $C_6$ haloalkyl, $C_1$ to $C_6$ alkoxy, nitro and cyano;
$R^2$ is chosen from the group consisting of hydrogen, $C_1$ to $C_6$ alkyl, $C_2$ to $C_6$ alkenyl, $C_2$ to $C_6$ alkoxyalkyl, $C_1$ to $C_6$ haloalkyl, acetyl, propionyl and $C_2$ to $C_6$ alkoxy carbonyl; and
G is chosen from the group consisting of hydroxy, mercapto, $C_1$ to $C_{10}$ alkoxy, $C_1$ to $C_{10}$ haloalkoxy, $C_2$ to $C_{10}$ alkenyloxy, $C_2$ to $C_{10}$ alkynyloxy, $C_1$ to $C_{10}$ alkylthio, $C_2$ to $C_{10}$ alkenylthio, $C_3$ to $C_7$ cycloalkoxy, $C_3$ to $C_7$ cycloalkoxy substituted with one or two $C_1$ to $C_4$ alkyl groups, phenoxy, phenylthio, benzyloxy, benzylthio, the group $C_1$ to $C_{10}$ alkoxy substituted with a $C_1$ to $C_6$ alkoxy group, the groups phenoxy, phenylthio, benzyloxy and benzylthio wherein in each group the phenyl ring is substituted with one or two substituents chosen from the group consisting of halogen, nitro, cyano, $C_1$ to $C_6$ alkyl, $C_1$ to $C_6$ haloalkyl and $C_1$ to $C_6$ alkoxy, the group OM wherein M is the cation of an inorganic or organic base, the group $-NHSO_2R^3$ wherein $R^3$ is chosen from $C_1$ to $C_{10}$ alkyl and $C_1$ to $C_{10}$ haloalkyl, and the group $-NR^4R^5$ wherein $R^4$ and $R^5$ are independently chosen from the group consisting of hydrogen, $C_1$ to $C_6$ alkyl, phenyl and benzyl or $R^4$ and $R^5$ together form a heterocyclic ring.

3. A compound according to claim 1 wherein:
A, B and D are independently chosen from the group consisting of hydrogen, halogen, nitro, cyano, phenyl, $C_1$ to $C_6$ alkyl and $C_1$ to $C_6$ haloalkyl;

E and V are independently chosen from hydrogen and halogen;
$R^1$ is chosen from the group consisting of hydrogen, $C_1$ to $C_6$ alkyl, $C_1$ to $C_6$ alkanoyl and benzyl;
$R^2$ is chosen from hydrogen and $C_1$ to $C_6$ alkyl; and
G is chosen from hydroxy, $C_1$ to $C_{10}$ alkoxy, $C_2$ to $C_{10}$ alkenyloxy, $C_2$ to $C_{10}$ alkynyloxy, $C_3$ to $C_7$ cycloalkoxy, benzyloxy, $C_1$ to $C_{10}$ alkoxy substituted with a substituent chosen from amino, ammonio, N-($C_1$ to $C_6$ alkyl)-amino, N,N-di($C_1$ to $C_6$ alkyl)amino and N,N,N-tri ($C_1$ to $C_6$ alkyl)ammonio and the group OM wherein M is an alkali metal or alkaline earth metal ion.

4. A compound according to claim 1 or claim 3 wherein:
A, D, E and V are hydrogen;
B is chosen from the group consisting of chlorine, bromine, iodine, nitro, phenyl and trifluoromethyl;
$R^1$ is chosen from hydrogen, methyl and ethyl;
$R^2$ is methyl; and
G is chosen from hydroxy, $C_1$ to $C_6$ alkoxy, $C_2$ to $C_6$ alkenyloxy, $C_2$ to $C_6$ alkynyloxy and $C_1$ to $C_6$ alkoxy substituted with a group chosen from N,N-di($C_1$ to $C_6$ alkyl)amino and N,N,N-tri($C_1$ to $C_6$ alkyl)ammonio.

5. A compound according to any one of claims 1, 3 or 4 wherein:
A, D, E and V are hydrogen;
B is chosen from the group consisting of chlorine, bromine, iodine and trifluoromethyl;
$R^1$ is chosen from methyl and ethyl;
$R^2$ is methyl; and
G is chosen from $C_1$ to $C_6$ alkoxy, 2-(N,N-dimethylamino)-ethoxy and 2-(N,N,N-trimethylammonio)ethoxy iodide.

6. A compound according to any one of claims 1 to 5 inclusive wherein:

A, D, E and V are hydrogen;
B is chosen from the group consisting of chlorine, bromine, iodine and trifluoromethyl;
$R^1$ is chosen from methyl and ethyl;
$R^2$ is methyl; and
G is $C_1$ to $C_6$ alkoxy.

7. A compound according to any one of claims 1 to 6 inclusive chosen from the group consisting of:
methyl 2-/4-(5-chloro-N-methyl-2-pyrimidylamino)phenoxy/-propionate;
methyl 2-/4-(5-bromo-N-methyl-2-pyrimidylamino)phenoxy/-propionate;
methyl 2-/4-(5-iodo-N-methyl-2-pyrimidylamino)phenoxy/-propionate;
methyl 2-/4-(N-methyl-5-trifluoromethyl-2-pyrimidylamino)-phenoxy/propionate;
methyl 2-/4-(5-bromo-N-ethyl-2-pyrimidylamino)phenoxy/-propionate; and
methyl 2-/4-(N-ethyl-5-trifluoromethyl-2-pyrimidylamino)-phenoxy/propionate.

8. A compound of formula VII

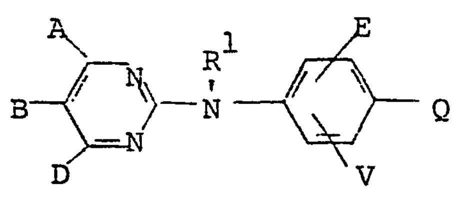

VII

wherein A, B, D, E, V, and $R^1$ are as defined according to any one of claims 1 to 7 inclusive and Q is chosen from hydroxy and $C_1$ to $C_6$ alkoxy.

9. A herbicidal composition comprising as active ingredient a compound as defined according to any one of claims 1 to 7 inclusive and a carrier therefor.

10. A composition according to claim 9 wherein said composition is in the form of a liquid and comprises a surface active agent.

11. A composition according to claim 9 wherein said composition is in the form of a powder.

12. A dilute composition according to any one of claims 9 to 11 inclusive which comprises from 0.01 to 2% by weight of active ingredient.

13. A concentrated composition according to any one of claims 9 to 11 inclusive which comprises from 20 to 90% by weight of active ingredient.

14. A process for severely damaging or killing unwanted plants which process comprises applying to said plants, or to the growth medium of said plants, an effective amount of a compound as defined according to any one of claims 1 to 7 inclusive or an effective amount of a composition as defined according to any one of claims 9 to 13 inclusive.

15. A process for selectively controlling the growth of monocotyledonous weeds in dicotyledonous crops which process comprises applying to said crop, or to the growth medium of said crop, a compound as defined according to any one of claims 1 to 7 inclusive or a composition as defined according to any one of claims 9 to 13 inclusive in an amount sufficient to severely damage or kill the weeds but insufficient to substantially damage the crop.

16. A process according to claim 14 or claim 15 wherein the compound is applied at a rate in the range from 0.05 to 20 kilograms per hectare.

17. A process according to claim 16 wherein the rate

is in the range from 0.1 to 10 kilograms per hectare.

18. A process for the synthesis of a compound of formula I as defined according to any one of claims 1 to 7 inclusive which process comprises the condensation, in the presence of an alkaline material, of a 4-(2-pyrimidylamino)phenol of formula II with a compound of formula III wherein hal is chlorine, bromine or iodine.

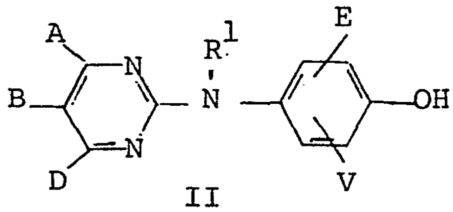

II

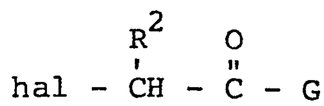

III

19. A process for the synthesis of a compound of formula I as defined according to any one of claims 1 to 7 inclusive which process comprises the condensation of a pyrimidine of formula IV, wherein L is a leaving group, with an aniline of formula V.

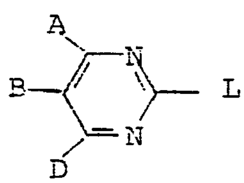

IV

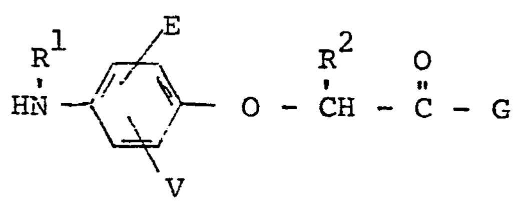

V

20. A process for the synthesis of a compound of formula I as defined according to any one of claims 1 to 7 inclusive which process comprises the following steps in sequence:

a) the condensation of a pyrimidine of formula IV, wherein L is a leaving group, with an aniline of formula VI, wherein Q is hydroxy or $C_1$ to $C_6$ alkoxy, to give a compound of formula VII;

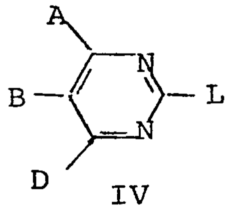

IV

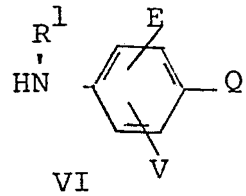

VI

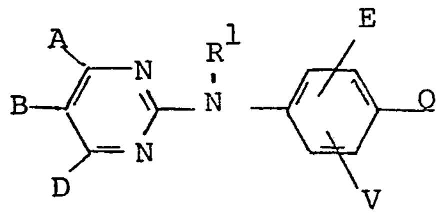

VII

b) the dealkylation of the compound of formula VII, wherein Q is $C_1$ to $C_6$ alkoxy, to give a phenol of formula II; and

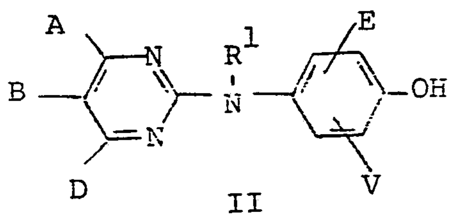

II

c) the condensation, in the presence of an alkaline material, of a compound of formula II obtained from step a) or step b) above with a compound of formula III wherein hal is chlorine, bromine or iodine.

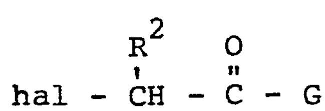

III

21. A compound of formula I as defined according to any one of claims 1 to 7 inclusive substantially as herein described with reference to any one of Examples 1 to 15 inclusive.

22. A compound of formula VII as defined according to claim 8 substantially as herein described with reference to any one of Examples 1 to 8 inclusive or Example 10.

23. A composition as defined according to any one of claims 9 to 13 inclusive substantially as herein described with reference to any one of Examples 16 to 19 inclusive.

24. A process as defined according to any one of claims 14 to 17 inclusive substantially as herein described with reference to any one of Examples 17 to 19 inclusive.

25. A process as defined according to any one of claims 18 to 20 inclusive substantially as herein described with reference to any one of Examples 1 to 15 inclusive.

DATED this day of 1979

ICI AUSTRALIA LIMITED